# EUROPEAN PATENT APPLICATION

(11) **EP 4 372 492 A1**
(43) Date of publication of application: **22.05.2024**
(21) Application number: 23208415.2
(22) Date of filing: 07.11.2023
(51) Int. Cl.: G05B 15/02

(54) **ENVIRONMENTAL CONTROL SYSTEM AND CARRIER MEDIUM**

(30) Priority: 18.11.2022 JP 2022185030; 29.09.2023 JP 2023170784
(71) Applicant: Ricoh Company, Ltd., Tokyo 143-8555 (JP)
(72) Inventor: Kimura, Yuta, Tokyo, 143-8555 (JP); Murata, Haruki, Tokyo, 143-8555 (JP)
(74) Representative: Marks & Clerk LLP

(57) **Abstract**

An environmental control system for controlling an environment of a space in which a user performs an activity includes a user information acquisition unit (34) to receive position information and direction information of a user. The environmental control system further includes an environmental control unit (36, 37) to control an environment of a space in which the user performs an activity, in accordance with the position information and the direction information of the user.

## Description

### BACKGROUND

### Technical Field

Embodiments of the present disclosure relate to an environmental control system and a carrier medium.

### Related Art

Currently, telework or remote work is widespread, and a more efficient way of working and creative output are desired for collaborative work performed by employees in an office. An approach to promote the interaction among employees in, for example, an office so as to efficiently produce more creative output is detecting and analyzing activity information of the employees in the office and feeding back the result to the environment.

Some technologies to support communication between multiple persons in the same space are known in the art (for example, see Japanese Translation of PCT International Application Publication No. JP-T-2018-512607). Japanese Translation of PCT International Application Publication No. JP-T-2018-512607 discloses an information processing apparatus that detects the positions of users in a space and displays an image to support the communication between the users.

However, in the related art, environmental control in accordance with the positions and directions of the users is not performed. For example, the related art does not propose environmental control in accordance with the positions and directions of users to help a user working in a space to efficiently communicate with another user in the space or help one or more users in a space to work efficiently. Examples of environmental control include adjusting tone and outputting images.

### SUMMARY

In an embodiment, an environmental control system for controlling an environment of a space in which a user performs an activity includes a user information acquisition unit to receive position information and direction information of a user. The environmental control system further includes an environmental control unit to control an environment of a space in which the user performs an activity, in accordance with the position information and the direction information of the user.

In another embodiment, a carrier medium carries computer readable codes which, when executed by a computer system, cause the computer system to carry out a method for controlling an environment of a space in which a user performs an activity. The method includes acquiring position information and direction information of a user in a space in which a user performs an activity, and controlling an environment of the space in accordance with the position information and the direction information of the user.

According to the embodiments of the present disclosure, environmental control according to the position and the direction of a user can be achieved.

### BRIEF DESCRIPTION OF THE DRAWINGS

A more complete appreciation of embodiments of the present disclosure and many of the attendant advantages and features thereof can be readily obtained and understood from the following detailed description with reference to the accompanying drawings, wherein:
FIG. 1 is a diagram illustrating environmental control provided for a user who performs some activities in a space such as a meeting room according to one embodiment of the present disclosure;
FIG. 2 is a block diagram illustrating a configuration of an environmental control system according to one embodiment of the present disclosure;
FIG. 3 is a diagram illustrating a meeting room according to one embodiment of the present disclosure;
FIG. 4 is a block diagram illustrating a hardware configuration of a computer according to one embodiment of the present disclosure;
FIG. 5 is a block diagram illustrating a hardware configuration of a smartphone according to one embodiment of the present disclosure;
FIG. 6 is a diagram illustrating a hardware configuration of an activity sensor according to one embodiment of the present disclosure;
FIG. 7 is a block diagram illustrating functional configurations of the activity sensor illustrated in FIG. 6 and an information processing system according to Embodiment 1 of the present disclosure;
FIG. 8 is a diagram illustrating a data structure of activity information according to one embodiment of the present disclosure;
FIG. 9 is a diagram illustrating a data structure of psychological safety information according to one embodiment of the present disclosure;
FIG. 10 is a diagram illustrating a data structure of tone and image information according to one embodiment of the present disclosure;
FIG. 11 is a diagram illustrating the direction of a user according to one embodiment of the present disclosure;
FIGS. 12A, 12B, and 12C are diagrams each illustrating the grouping of users according to one embodiment of the present disclosure;
FIG. 13 is a diagram illustrating a method of determining whether users face each other when another user is present, according to one embodiment of the present disclosure;
FIG. 14 illustrates two users facing each other;
FIG. 15 is a flowchart of a process of outputting a tone and an image corresponding to a psychological safety level, performed by the information processing system illustrated in FIG. 7;
FIG. 16 is a diagram illustrating changes in the psychological safety level between users day by day, determined by the information processing system illustrated in FIG. 7;
FIG. 17 is a table presenting percentages of time during which a listener faces a speaker in the team, calculated by a psychological safety estimation unit illustrated in FIG. 7;
FIG. 18 is a flowchart of a process of scoring the likelihood of occurrence of psychological safety of a team based on the percentages of time during which a listener faces a speaker, performed by the psychological safety estimation unit illustrated in FIG. 7;
FIG. 19 is a table presenting the frequencies of nodding of the listeners to the speaker in a team, calculated by the psychological safety estimation unit illustrated in FIG. 7;
FIG. 20 is a flowchart of a process of scoring the likelihood of occurrence of psychological safety of a team based on the frequencies of nodding of a listener to a speaker, performed by the psychological safety estimation unit illustrated in FIG. 7;
FIG. 21 is a diagram illustrating the creation of minutes in which a conversation partner is identified, according to one embodiment of the present disclosure;
FIG. 22 is a flowchart of a process of creating minutes in which a conversation partner is identified, according to one embodiment of the present disclosure;
FIG. 23 is a diagram illustrating image display devices to which users face, according to one embodiment of the present disclosure;
FIG. 24 is a diagram illustrating the relation between the direction of a user and the surface of an image display device according to one embodiment of the present disclosure;
FIG. 25 is a flowchart of a process of guiding a user to an image display device, performed by a user guide unit illustrated in FIG. 7;
FIG. 26 is a diagram illustrating voice input to image display devices by users, according to one embodiment of the present disclosure;
FIG. 27 is a flowchart of a process of displaying user's speech data in a work area, performed by a second environmental control unit illustrated in FIG. 7;
FIGS. 28A to 28D are graphs each illustrating a method of evaluating individuality based on speech data, performed by an individuality evaluation unit illustrated in FIG. 7;
FIG. 29 is a flowchart of a process of scoring the individuality of a team, performed by the individuality evaluation unit illustrated in FIG. 7;
FIG. 30 is a block diagram illustrating a functional configuration of an information processing system according to Embodiment 2 of the present disclosure;
FIG. 31 is a diagram illustrating a method of determining an understanding level as a user state according to Embodiment 2 of the present disclosure;
FIG. 32 is a diagram illustrating a data structure of tone and image information according to Embodiment 2 of the present disclosure;
FIG. 33 is a flowchart of a process of displaying the understanding levels of users having a face-to-face conversation, performed by a second environmental control unit according to Embodiment 2 of the present disclosure;
FIG. 34 is a diagram illustrating a method of determining a concentration level as another user state, according to Embodiment 2 of the present disclosure;
FIG. 35 is a flowchart of a process of environmental control in accordance with a concentration level, according to Embodiment 2 of the present disclosure;
FIG. 36 is a diagram illustrating a method of displaying a cursor and usage of the cursor according to Embodiment 2 of the present disclosure;
FIG. 37 is a flowchart of the method of displaying a cursor and usage of the cursor according to Embodiment 2 of the present disclosure;
FIG. 38 is a diagram illustrating correspondence between an office layout and convenience according to Embodiment 2 of the present disclosure;
FIG. 39 is a flowchart of a process of presenting the conveniences of places in an office, according to Embodiment 2 of the present disclosure;
FIG. 40 is a diagram illustrating the determination of a place where meaningful communications are likely to be performed in an office, according to Embodiment 2 of the present disclosure;
FIG. 41 is a flowchart of a process of presenting a place where meaningful communications are performed in an office layout, according to Embodiment 2 of the present disclosure;
FIG. 42 is a diagram illustrating a system configuration of an information processing system that generates a virtual space according to Embodiment 3 of the present disclosure;
FIG. 43 is a diagram illustrating a system configuration of an information processing system that generates a virtual office as a virtual space, according to Embodiment 3 of the present disclosure;
FIG. 44 is a block diagram illustrating a hardware configuration of a display terminal according to Embodiment 3 of the present disclosure;
FIG. 45 is a block diagram illustrating a hardware configuration of a controller according to Embodiment 3 of the present disclosure;
FIG. 46 is a block diagram illustrating a functional configuration of an information processing system according to Embodiment 3 of the present disclosure;
FIG. 47 is a block diagram illustrating functional configurations of the display terminal illustrated in FIG. 44 and the controller illustrated in FIG. 45 according to Embodiment 3 of the present disclosure;
FIG. 48 is a block diagram illustrating in detail a functional configuration of a virtual space control unit according to Embodiment 3 of the present disclosure;
FIG. 49 is a flowchart of a process of outputting a tone and an image corresponding to a psychological safety level in a virtual space, performed by the information processing system illustrated in FIG. 46;
FIG. 50 is a flowchart of a process of creating minutes in which the conversation partner is identified in a virtual space, according to Embodiment 3 of the present disclosure;
FIG. 51 is a flowchart of a process of guiding an avatar to an image display device in a virtual space, performed by a user guide unit according to Embodiment 3 of the present disclosure;
FIG. 52 is a flowchart of a process of displaying the avatar's speech data in a work area, performed by a second environmental control unit according to Embodiment 3 of the present disclosure;
FIG. 53 is a flowchart of a process of displaying the understanding levels of avatars having a face-to-face conversation in a virtual space, performed by the second environmental control unit according to Embodiment 3 of the present disclosure;
FIG. 54 is a flowchart of a process of environmental control in a virtual space in accordance with the concentration level of a user, according to Embodiment 3 of the present disclosure;
FIG. 55 is a flowchart of a method of displaying a cursor and usage of the cursor in a virtual space, according to Embodiment 3 of the present disclosure;
FIG. 56 is a flowchart of a process of scoring the individuality of a team in a virtual space, performed by an individuality evaluation unit according to Embodiment 3 of the present disclosure;
FIG. 57 is a flowchart of a process of presenting the conveniences on an office layout, according to Embodiment 3 of the present disclosure; and
FIG. 58 is a flowchart of a process of presenting ease of communication on an office layout, according to Embodiment 3 of the present disclosure.

The accompanying drawings are intended to depict embodiments of the present disclosure and should not be interpreted to limit the scope thereof. The accompanying drawings are not to be considered as drawn to scale unless explicitly noted. Also, identical or similar reference numerals designate identical or similar components throughout the several views.

### DETAILED DESCRIPTION

In describing embodiments illustrated in the drawings, specific terminology is employed for the sake of clarity. However, the disclosure of this specification is not intended to be limited to the specific terminology so selected and it is to be understood that each specific element includes all technical equivalents that have a similar function, operate in a similar manner, and achieve a similar result falling within the scope of the appended claims.

Referring now to the drawings, embodiments of the present disclosure are described below. As used herein, the singular forms "a," "an," and "the" are intended to include the plural forms as well, unless the context clearly indicates otherwise.

A description is given below of an environmental control system and an environmental control method performed by the environmental control system according to embodiments of the present disclosure, with reference to the drawings.

### Embodiment 1

### Outline of Operation or Processing

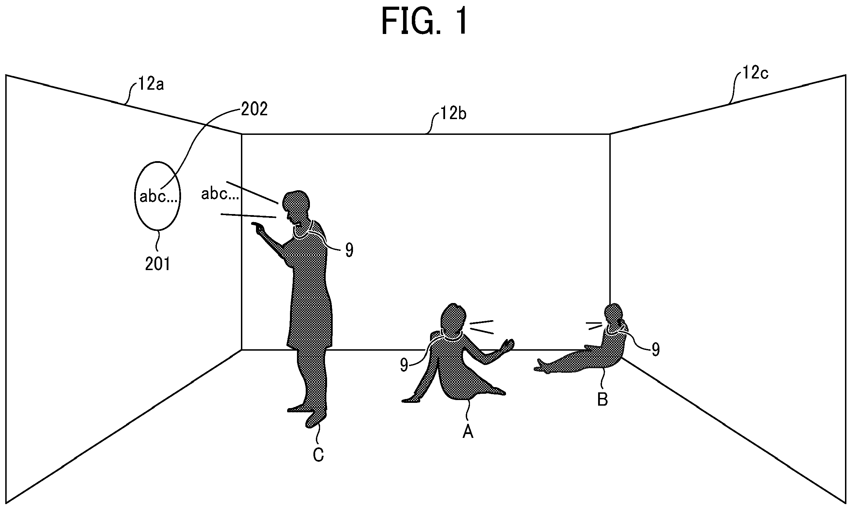

Referring to FIG. 1, a description is given of an outline of an operation or processing of the environmental control system according to Embodiment 1 of the present disclosure. FIG. 1 is a diagram illustrating environmental control provided for users performing activities in a space such as a meeting room. Each user carries an activity sensor 9, which will be described later. The activity sensor 9 constantly transmits measured activity information (such as the position, the direction, the behavior, the voice, and the heartbeat of the user) to an information processing system, which is, for example, a cloud-based system.

In the case illustrated in FIG. 1, the information processing system detects from the activity information that a user A faces a user B. The information processing system estimates or determines the levels of psychological safety of the users A and B by measuring the time during which the users A and B face each other. The time during which multiple persons face each other may be referred to as "face-to-face time" in the following description. The information processing system can output music and images in accordance with the level of psychological safety.

In the case illustrated in FIG. 1, the information processing system further detects, from the activity information, that a user C faces an image display device 12a. The user C may be moving or stay at a position. In other words, based on the position information included in the activity information of the user C, the information processing system detects that the user C directs the face or the body to a wall serving as a display of the image display device 12a or a screen generated by the image display device 12a. The image display devices 12a, 12b, and 12c may be collectively referred to as "image display devices 12" in the following description. The information processing system displays a work area 201 for the user C on the image display device 12a to guide the user C to the image display device 12a. In this case, the image display device 12a serves as an object to be used for the environmental control. Alternatively, the information processing system displays a work area 201 for the user C on the screen generated by the image display device 12a to guide the user C to the screen generated by the image display device 12a. The information processing system displays speech data 202 uttered by the user C in the work area 201. The information processing system can also display data handwritten or by the user C in the work area 201. Handwriting may include gesture-based input, motion tracking input or other touch-free input.

As described above, the environmental control system according to the present embodiment controls the environment such as sound and images in accordance with the position and the direction of the user. The present embodiment provides, for example, environmental control to promote conversation leading to creative output, or environmental control to help a user concentrate on work.

### Terminology

An "activity" refers to performing a certain movement or work. An "activity" is not limited to moving a body, but includes a mental activity. "Activity information" refers to information acquired by the activity sensor 9. However, "activity information" may be detected by, for example, a camera or a microphone disposed in a space.

A "space" refers to a place having a certain extent where one or more users can be present. A space is not limited to an indoor space but may be an outdoor space.

An "environment" refers to something that affects a user in some way in a space. An "environment" is something at least perceived by the user in five senses. In the present embodiment, a tone and an image are described as examples of the environment. Additionally or alternatively, the information processing system may perform the environmental control by vibration or smell.

"Environmental control" includes controlling a certain device so as to output something (such as video, image, sound, vibration, or smell) that affects any of the five senses of a user in a space.

"Position information" refers to information indicating the position of a user in a space. The position may be, for example, coordinates with respect to a reference point in the space. In the present embodiment, the position information is detected by ultra wideband (UWB). The position information may be represented by a latitude and a longitude detected by, for example, an indoor messaging system (IMES) or a global navigation satellite system (GNSS). In the present embodiment, the position information may be referred to simply as "position."

"Direction information" refers to information indicating the direction which the user faces in a space. The direction information is specified, for example, in a range of 0 to 360 degrees in the horizontal direction with respect to the north direction and in a range of 0 to 180 degrees in the elevation angle direction with respect to the zenith direction. The reference direction may be any direction. In the present embodiment, direction information may be referred to simply as "direction."

### System Configuration

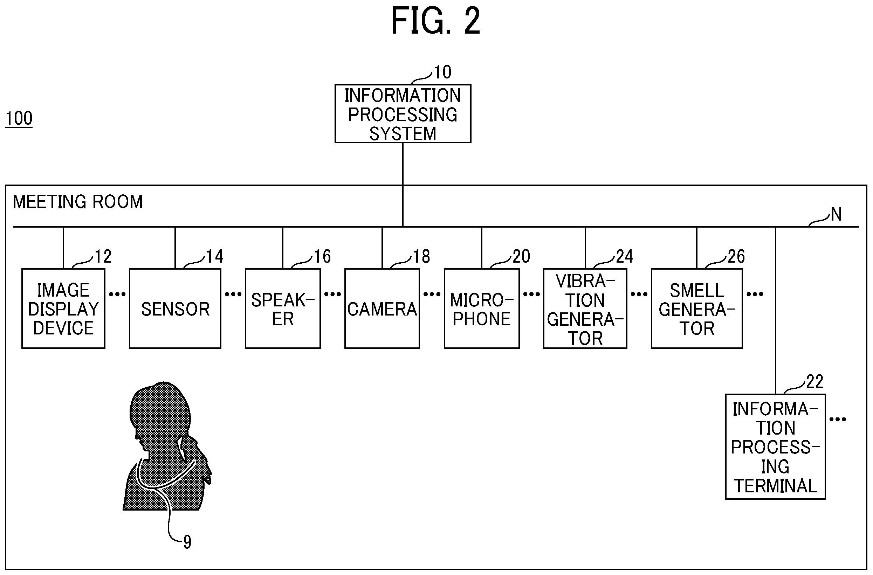

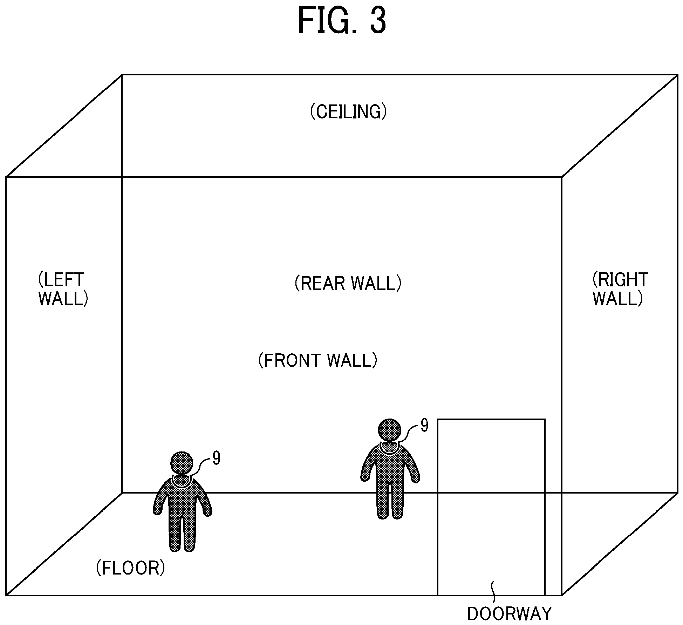

FIG. 2 is a schematic diagram illustrating a configuration of an environmental control system 100 according to the present embodiment. FIG. 3 is a diagram illustrating a meeting room according to the present embodiment. The environmental control system 100 of FIG. 2 includes an information processing system 10, an image display device 12, a sensor 14, a speaker 16, a camera 18, a microphone 20, and an information processing terminal 22 that are connected in a wired or wireless manner so as to communicate with each other via a network N such as the Internet or a local area network (LAN).

The meeting room is provided with the image display device 12, the sensor 14, the speaker 16, the camera 18, the microphone 20, and the information processing terminal 22. The meeting room may be provided with, for example, a temperature sensor, a humidity sensor, or an illuminance sensor that acquires at least a part of environment-dependent information and transmits the acquired information to the information processing system 10. Although FIG. 2 illustrates the information processing system 10 disposed outside the meeting room, the information processing system 10 may be disposed inside the meeting room.

For example, a user who enters a meeting room carries the activity sensor 9 that transmits radio waves such as a beacon. The sensor 14 in the meeting room receives the radio waves transmitted from the activity sensor 9 of the user in the meeting room as a signal for detecting the position information of the user and transmits the signal to the information processing system 10. The sensor 14 can be any sensor having a positioning system that outputs a signal for detecting the position information of the user.

The activity sensor 9 at a target (i.e., the user) to be measured includes, for example, two acceleration and angular velocity sensors, a microphone, and a vital sensor, and has a shape like a necklace to be worn around the neck of the user. The microphone of the activity sensor 9 receives the voice of the user wearing the activity sensor 9. The vital sensor acquires vital data from the user. The activity sensor 9 determines the behavior of the user based on signals detected by two or more acceleration and angular velocity sensors and values of the signals relative to a reference value.

The activity sensor 9 may be a dedicated sensor, a smart watch, a smartphone, or, for example, any of various types of BLUETOOTH LOW ENERGY (BLE) sensor. The information processing system 10 detects the position information of the users in the meeting room based on the signals for detecting the position information of the users transmitted from one or more sensors 14. The activity sensor 9 described above serves as a transmitter. The transmitter is not limited to the activity sensor 9 and may be any device that transmits a signal for detecting the position information of the user.

The information processing terminal 22 is a device operated by the user in the meeting room. Examples of the information processing terminal 22 include a laptop personal computer (PC), a mobile phone, a smartphone, a tablet communication terminal, a game console, a personal digital assistant (PDA), a digital camera, a wearable PC, a desktop PC, and a device dedicated to the meeting room. The information processing terminal 22 may be carried in the meeting room by the user. Alternatively, the meeting room may be provided with the information processing terminal 22.

The information processing terminal 22 may be a target to be measured by a positioning system. For example, the sensor 14 in the meeting room may receive radio waves transmitted from the activity sensor 9 of the information processing terminal 22 and transmit the received radio waves to the information processing system 10. For example, the sensor 14 transmits, to the information processing system 10, the signal for detecting the position information indicating the relative positions in the meeting room as illustrated in FIG. 3, of the user who operates the information processing terminal 22. The activity sensor 9 may be built in the information processing terminal 22 or may be in any other suitable form. The information processing terminal 22 may include a sensor that measures the heartbeat of the user, and may transmit, to the information processing system 10, the measured heartbeat of the user.

The camera 18 in the meeting room captures an image in the meeting room and transmits the captured image data to the information processing system 10 as an output signal. For example, the camera 18 is a video camera of KINECT. The video camera of KINECT serves as a video camera that includes a range image sensor, an infrared sensor, and an array microphone. When such a video camera including a range image sensor, an infrared sensor, and an array microphone is used, the motion and the posture of the user are recognized.

The microphone 20 in the meeting room converts the voice of each user into an electrical signal. The microphone 20 transmits the electrical signal converted from the voice of the user to the information processing system 10 as an output signal. In alternative to or in addition to the microphone 20 in the meeting room, a microphone of the information processing terminal 22 may be used.

The speaker 16 in the meeting room converts an electrical signal into a physical signal and outputs sound such as ambient sound. The speaker 16 outputs the sound such as the ambient sound under the control of the information processing system 10. In alternative to or in addition to the speaker 16 in the meeting room, a speaker of the information processing terminal 22 may be used. The microphone 20 in the meeting room and the microphone of the information processing terminal 22 serve as input devices. The speaker 16 in the meeting room and the speaker of the information processing terminal 22 serve as output devices.

In addition to the above-described devices illustrated in FIG. 2, other devices such as a vibration generator 24 to generate vibration by, for example, a motor, or a smell generator 26 to generate smell may be disposed in the meeting room. The vibration generator 24 includes a vibration motor, and a weight called a vibrator is coupled to a shaft that rotates. In the vibrator, a coupling portion with a rotation shaft is deviated from the center of gravity. When the vibrator rotates, the vibrator vibrates due to eccentricity. As this vibration propagates to the surroundings, the vibrator serves as a vibration source. The smell generator 26 may be of any type and, for example, of an air gun type or a blower type.

Two or more of the devices illustrated in FIG. 2 (i.e., the image display device 12, the sensor 14, the speaker 16, the camera 18, the microphone 20, the information processing terminal 22, the activity sensor 9, the vibration generator 24, and the smell generator 26) may be integrated into a single device.

The meeting room is provided with the multiple image display devices 12, such as a projector. The image display devices 12 can display images on the sides partitioning the meeting room as illustrated in FIG. 3 under the control of the information processing system 10. The sides partitioning the meeting room includes, for example, a front wall, a rear wall, a right wall, a left wall, a floor, and a ceiling. Similar to the position information, the position of each wall is specified by coordinates relative to a reference point (origin) that is the contact point between the end of a wall and the end of another wall. The projector can detect a user's operation (such as handwriting) on the wall by a known method. For example, the image display device 12 is a flat panel display such as a liquid crystal display or an organic electro-luminescence (EL) display. The flat panel display is preferably a touch panel and preferably has a large screen. The image display device 12 may be an electronic whiteboard having a flat panel display. The electronic whiteboard may be embedded in a wall or may be disposed in front of the wall. The image display device 12 serves as a display device that displays an image and may be any display device that has at least a function of displaying an image.

The shape of the meeting room illustrated in FIG. 3 is an example, and the meeting room can have any other shape. One or some of the sides (such as the side walls, the floor, and the ceiling) of the meeting room are not necessarily partitioned from the space outside the meeting room. In other words, a part of the sides of the meeting room may be open, not partitioned. The meeting room serves as the same space in which multiple users are present. Examples of the meeting room include various spaces such as a room for seminars or lectures, a meeting space, an event space, a children's room, a children's center, a classroom, a hospital, an amusement arcade, and an amusement park. As described above, the space described in the present embodiment is a concept including a place or a room where multiple users are present.

The information processing system 10 includes one or more information processing apparatuses. As will be described later, the information processing system 10 outputs the ambient sound or image suitable for interaction (for example, conversations and meetings) between the users in the meeting room, based on, for example, the position information of the users detected by the signal transmitted from the sensor 14, the output signal from the camera 18, and the output signal from the microphone 20.

The configuration of the information processing system 10 according to the present embodiment is not limited to that illustrated in FIG. 2. The information processing system 10 may be implemented by a single computer or multiple computers, or may be implemented by a cloud service. Examples of the information processing system 10 include a projector, a display device having an electronic whiteboard function, an output device such as digital signage, a head-up display (HUD) apparatus, an industrial machine, an image-capturing device, a sound collecting device, a medical device, a networked home appliance, a connected car, a laptop PC, a mobile phone, a smartphone, a tablet communication terminal, a game console, a PDA, a digital camera, a wearable PC, and a desktop PC.

### Hardware Configuration

### Hardware Configuration of Computer

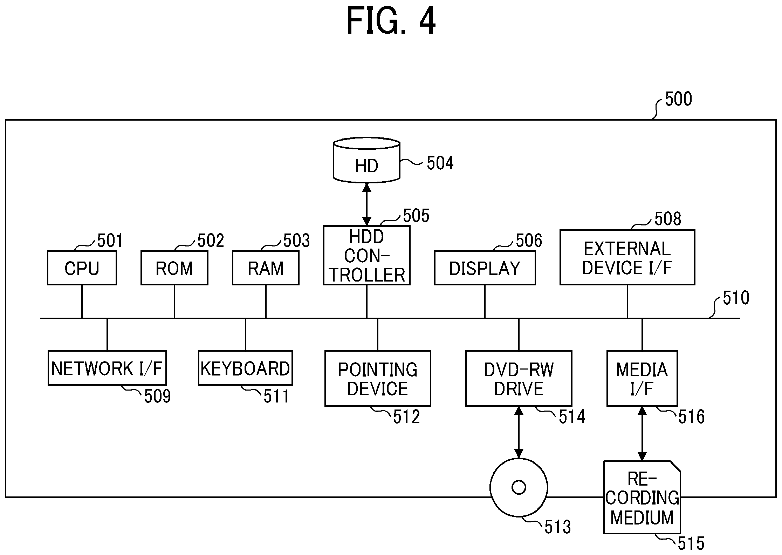

The information processing system 10 is implemented by, for example, a computer 500 having a hardware configuration illustrated in FIG. 4. In a case that the information processing terminal 22 is a PC, the information processing terminal 22 is implemented by the computer 500 having the hardware configuration illustrated in FIG. 4.

FIG. 4 is a block diagram illustrating a hardware configuration of a computer according to the present embodiment. As illustrated in FIG. 4, the computer 500 includes a central processing unit (CPU) 501, a read-only memory (ROM) 502, a random access memory (RAM) 503, a hard disk (HD) 504, a hard disk drive (HDD) controller 505, a display 506, an external device interface (I/F) 508, a network I/F 509, a data bus 510, a keyboard 511, a pointing device 512, a digital versatile disk rewritable (DVD-RW) drive 514, and a media I/F 516.

The CPU 501 controls the entire operation of the computer 500. The ROM 502 stores programs, such as an initial program loader (IPL), for driving the CPU 501. The RAM 503 is used as a work area for the CPU 501. The HD 504 stores various kinds of data such as a program. The HDD controller 505 controls the reading and writing of various data from and to the HD 504 under the control of the CPU 501.

The display 506 displays various information such as a cursor, a menu, a window, a character, and an image. The external device I/F 508 is an interface for connecting various external devices. Examples of the external devices in this case include, but are not limited to, a universal serial bus (USB) memory and a printer. The network I/F 509 is an interface for performing data communication via the network N. Examples of the data bus 510 include, but are not limited to, an address bus and a data bus that electrically connect the components such as the CPU 501 with one another.

The keyboard 511 is a kind of input device provided with multiple keys for inputting, for example, characters, numerals, and various instructions. The pointing device 512 is a kind of input device used to, for example, select various instructions, execute various instructions, select a target for processing, and move a cursor. The DVD-RW drive 514 reads and writes various data from and to a DVD-RW 513 serving as a removable recording medium according to the present embodiment. The DVD-RW may be, for example, a digital versatile disc-recordable (DVD-R). The media I/F 516 controls the reading or writing (storing) of data from or to a recording medium 515 such as a flash memory.

### Smartphone

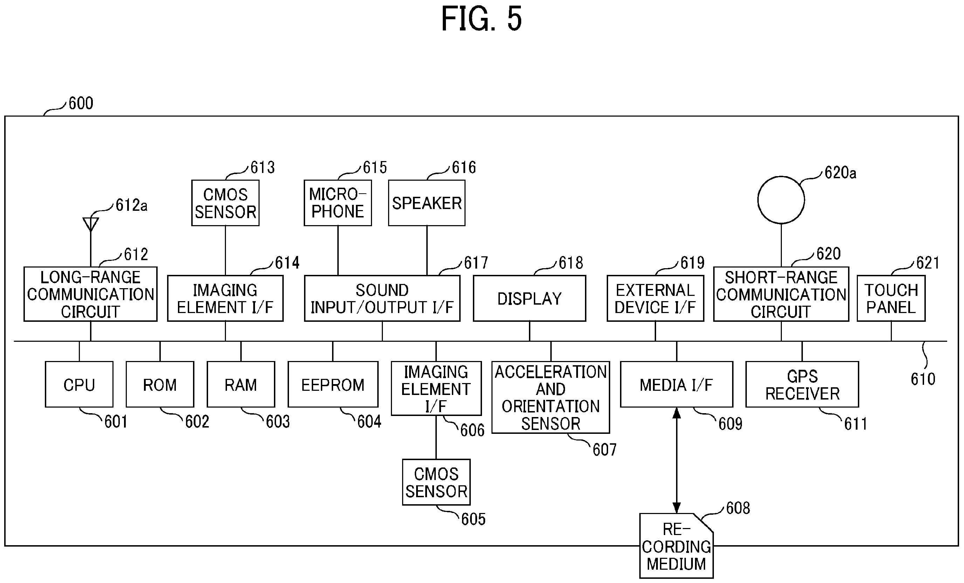

The information processing terminal 22 is implemented by, for example, a smartphone 600 having a hardware configuration illustrated in FIG. 5. Even when the information processing terminal 22 is not a smartphone but is, for example, a laptop PC, a mobile phone, a smartphone, a tablet communication terminal, a game console, a PDA, a digital camera, a wearable PC, a desktop PC, or a device dedicated to a meeting room, the information processing terminal 22 may be implemented by a hardware configuration similar to the hardware configuration as illustrated in FIG. 5. Further, a part of the hardware configuration illustrated in FIG. 5 may be excluded, or another hardware component may be added to the hardware configuration illustrated in FIG. 5.

FIG. 5 is a block diagram illustrating a hardware configuration of the smartphone according to the present embodiment. As illustrated in FIG. 5, a smartphone 600 includes a CPU 601, a ROM 602, a RAM 603, an electrically erasable and programmable ROM (EEPROM) 604, a complementary metal oxide semiconductor (CMOS) sensor 605, an imaging element I/F 606, an acceleration and orientation sensor 607, a media I/F 609, and a global positioning system (GPS) receiver 611.

The CPU 601 controls the entire operation of the smartphone 600. The ROM 602 stores, for example, a program used by the CPU 601 and a program such as an IPL for driving the CPU 601. The RAM 603 is used as a work area for the CPU 601. The EEPROM 604 reads or writes various data such as a control program for the smartphone under the control of the CPU 601.

The CMOS sensor 605 is a kind of built-in image-capturing device to capture an image of an object (for example, a self-image of a user operating the smartphone 600) under the control of the CPU 601, to obtain image data. The CMOS sensor 605 may be an image-capturing device such as a charge-coupled device (CCD) sensor. The imaging element I/F 606 is a circuit that controls the driving of the CMOS sensor 605. Examples of the acceleration and orientation sensor 607 include various types of sensors such as an electromagnetic compass for detecting geomagnetism, a gyrocompass, and an accelerometer.

The media I/F 609 controls the reading or writing (storing) of data from or to a recording medium 608 such as a flash memory. The GPS receiver 611 receives a GPS signal from a GPS satellite.

The smartphone 600 further includes a long-range communication circuit 612, a CMOS sensor 613, an imaging element I/F 614, a microphone 615, a speaker 616, a sound input/output I/F 617, a display 618, an external device I/F 619, a short-range communication circuit 620, an antenna 620a for the short-range communication circuit 620, and a touch panel 621.

The long-range communication circuit 612 is a circuit for communicating with other devices through the network N. The CMOS sensor 613 is a kind of built-in image-capturing device to capture an image of a subject under the control of the CPU 601, to obtain image data. The imaging element I/F 614 is a circuit that controls the driving of the CMOS sensor 613. The microphone 615 is a built-in circuit that converts sound including voice into an electrical signal. The speaker 616 is a built-in circuit that generates sound such as ambient sound, music, or voice by converting an electrical signal into physical vibration.

The sound input/output I/F 617 is a circuit that processes the input and output of sound signals between the microphone 615 and the speaker 616 under the control of the CPU 601. The display 618 is a kind of display device to display, for example, an image of a subject and various icons. Examples of the display 618 include a liquid crystal display and an organic EL display.

The external device I/F 619 is an interface for connecting various external devices. The short-range communication circuit 620 is a communication circuit that is compliant with Near Field Communication (NFC) or BLUETOOTH, for example. The touch panel 621 is a kind of input device to enable a user to operate the smartphone 600 by touching or pressing a screen of the display 618.

The smartphone 600 further includes a bus line 610. The bus line 610 is, for example, an address bus or a data bus for electrically connecting the components such as the CPU 601 illustrated in FIG. 5 to one another.

### Activity Sensor

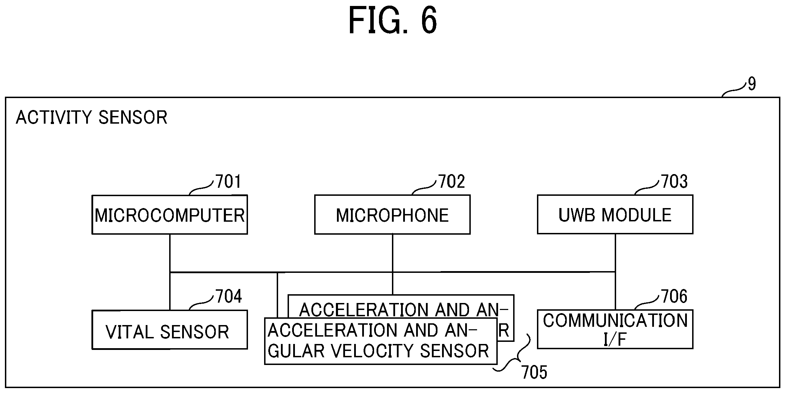

FIG. 6 is a block diagram illustrating a hardware configuration of the activity sensor 9 according to the present embodiment. As illustrated in FIG. 6, the activity sensor 9 includes a microcomputer 701, a microphone 702, a UWB module 703, a vital sensor 704, two acceleration and angular velocity sensors 705, and a communication I/F 706.

The microcomputer 701 has functions of components, such as a CPU, a ROM, a RAM, and an EEPROM, of a general-purpose computer. The microphone 702 is a built-in circuit that converts sound including voice into an electrical signal. The UWB module 703 periodically transmits radio waves having short and sharp rectangular waveforms (pulses). Although the communication range thereof is as short as about 10 meters, UWB module 703 enables high-speed communication with reduced power consumption. The sensor 14 detects the direction and the distance of the activity sensor 9 with high accuracy with an error of about several centimeters by receiving radio waves of a rectangular waveform (pulse). The vital sensor 704 detects vital data of the user. The vital data is an index such as a heartbeat, a pulse, or blood pressure which indicates that a person is alive. The acceleration and angular velocity sensor 705 is also referred to as an inertial sensor, and detects accelerations in three axial directions generated in the user and angular velocities of rotation about the three axes. The acceleration and angular velocity sensors 705 are disposed one on each of the front side and rear side of the user's neck. The microcomputer 701 determines the direction of the user, for example, by integrating the angular velocity. Further, the microcomputer 701 determines the behavior of the user on the basis of the acceleration or the angular velocity detected by the two acceleration and angular velocity sensors 705. The communication I/F 706 connects to a network such as a wireless LAN by, for example, BLUETOOTH, to transmit the activity information detected by the activity sensor 9 to the sensor 14 or the information processing system 10.

### Functional Configuration

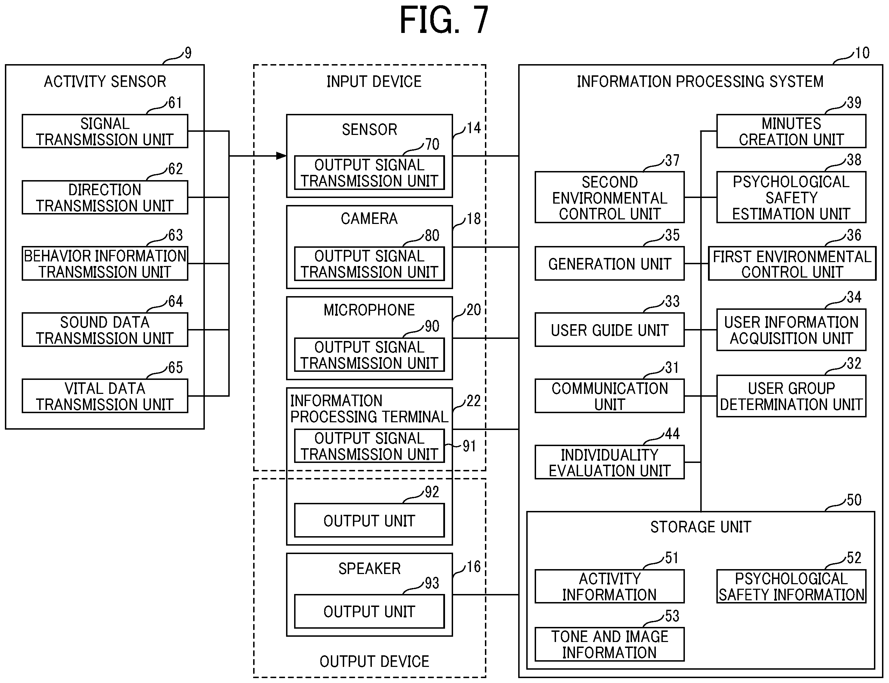

The environmental control system 100 according to the present embodiment is implemented, for example, by a functional configuration illustrated in FIG. 7. FIG. 7 is a block diagram illustrating a functional configuration of the information processing system 10 and the activity sensor 9 according to the present embodiment. FIG. 7 illustrates the functional configuration used for describing to the present embodiment, and the information processing system 10 may include other functional components.

### Activity Sensor

The activity sensor 9 includes a signal transmission unit 61, a direction transmission unit 62, a behavior information transmission unit 63, a sound data transmission unit 64, and a vital data transmission unit 65. These functional units of the activity sensor 9 implement functions or means as the microcomputer 701 (illustrated in FIG. 6) executes instructions included in one or more programs installed on the activity sensor 9.

The signal transmission unit 61 periodically transmits to a sensor device a signal by radio waves called UWB. The signal includes a user identifier (ID), and the sensor 14 can detect the position of the user with the user identified. The position information of the user is represented by two-dimensional coordinates (x, y) based on a predetermined position in the meeting room. The position information of the user is specified by the coordinates with respect to a reference point (origin). For example, the reference point is a point of contact between the end of a wall and the end of another wall of the meeting room or the center of the meeting room.

The direction transmission unit 62 transmits the direction of the user detected by the microcomputer 701 to the sensor 14. The direction is specified, for example, in a range of 0 to 360 degrees in the horizontal direction with respect to the north direction and in a range of 0 to 180 degrees in the elevation angle direction with respect to the zenith direction. The direction may be calculated from the angular velocity by the information processing system 10 instead of being detected by the microcomputer 701.

The behavior information transmission unit 63 transmits the behavior information of the user detected by the microcomputer 701 to the sensor 14. Examples of the behavior include a nod, a head tilt, a face-up posture, an inclined posture, the direction of a face, and the stationary degree of a face. It is assumed that the correspondence between the behavior information and the acceleration and the angular velocity is obtained in advance by machine learning, for example. In other words, the behavior information transmission unit 63 outputs the behavior information corresponding to the acceleration and the angular velocity included in the activity information. The behavior information may be calculated by the information processing system 10 from acceleration and the angular velocity instead of being detected by the microcomputer 701.

Machine learning is a technique that enables a computer to acquire human-like learning ability. Machine learning refers to a technology in which a computer autonomously generates an algorithm to be used for determination such as data identification from training data loaded in advance and applies the generated algorithm to new data to make a prediction. The method for machine learning may be any suitable method such as one of supervised learning, unsupervised learning, semi-supervised learning, reinforcement learning, and deep learning, and two or more of these learning methods may be combined. Examples of machine learning techniques include perceptron, deep learning, support-vector machine, logistic regression, naive Bayes, decision tree, and random forests, and are not limited the techniques described in the present embodiment.

For example, deep learning is an algorithm in which XYZ is predicted based on input data ABC, and then weights between neural networks are adjusted by back propagation, in order to reduce an error from teacher data. Gradient boosting using decision trees is an algorithm that causes multiple weak prediction models to independently perform learning using a gradient method, integrates prediction results by the multiple weak prediction models using, for example, the majority vote or the average, and outputs the integrated prediction result as a prediction result of the whole (a strong prediction model).

The sound data transmission unit 64 transmits sound data received by the microphone 702 to the sensor 14.

The sound data is converted into digital signals. The information processing system 10 performs speech recognition on the sound data and converts the sound data into speech data (text data).

The vital data transmission unit 65 transmits the vital data acquired by the vital sensor 704 to the sensor 14. Examples of the vital data include the heartbeat, the pulse, the upper and lower blood pressures, the body temperature, the saturated oxygen concentration, the amount of perspiration, and the subjective symptom (consciousness level) of the user. The vital data may include any information detectable from the user.

Although the activity sensor 9 transmits the activity information to the sensor 14 in the description given above, alternatively, the activity sensor 9 may directly transmit the activity information to the information processing system 10.

### Input Device and Output Device

The sensor 14 includes an output signal transmission unit 70. The camera 18 includes an output signal transmission unit 80. The microphone 20 includes an output signal transmission unit 90. The information processing terminal 22 includes an output signal transmission unit 91 and an output unit 92. The speaker 16 includes an output unit 93.

The output signal transmission unit 70 of the sensor 14 transmits the activity information received from the activity sensor 9 to the information processing system 10. In other words, the output signal transmission unit 70 transmits the respective position information of the multiple users in the meeting room to the information processing system 10 together with the user ID. Further, the sensor 14 transmits the direction, the behavior information, the speech data converted from the sound data, and the vital data received from the activity sensor 9 to the information processing system 10 as output signals.

The output signal transmission unit 80 of the camera 18 transmits to the information processing system 10 an image-capturing result obtained by capturing an image of the inside of the meeting room as an output signal. The output signal transmission unit 90 of the microphone 20 transmits to the information processing system 10 electrical signals converted from the voices of the multiple users in the meeting room as output signals.

The output signal transmission unit 91 of the information processing terminal 22 transmits to the information processing system 10 an electrical signal converted by the microphone 615 from the voice of the user operating the information processing terminal 22 as an output signal. The output unit 92 of the information processing terminal 22 outputs sound such as an ambient sound in accordance with the sound data received from the information processing system 10. The output unit 93 of the speaker 16 outputs sound such as an ambient sound in accordance with the sound data received from the information processing system 10.

The output signal transmission units 70, 80, 90, and 91 illustrated in FIG. 7 serve as input devices. The output units 92 and 93 serve as output devices.

### Information Processing System

The information processing system 10 includes a communication unit 31, a user group determination unit 32, a user guide unit 33, a user information acquisition unit 34, a generation unit 35, a first environmental control unit 36, a second environmental control unit 37, a psychological safety estimation unit 38, a minutes creation unit 39, an individuality evaluation unit 44, and a storage unit 50. The storage unit 50 stores activity information 51, psychological safety information 52, and tone and image information 53, which will be described later. The functional units of the information processing system 10 are implemented as the CPU 501 illustrated in FIG. 4 executes instructions included in one or more programs installed on the information processing system 10. The storage unit 50 is built in, for example, the RAM 503 or HD 504 illustrated in FIG. 4.

The communication unit 31 of the information processing system 10 receives the activity information detected by the activity sensor 9 from the output signal transmission unit 70 of the sensor 14. The communication unit 31 receives the image-capturing result obtained by capturing the image of the inside of the meeting room as the output signal from the output signal transmission unit 80 of the camera 18. The communication unit 31 receives the electrical signals converted from the voices of the multiple users in the meeting room as the output signals from the output signal transmission unit 90 of the microphone 20. The communication unit 31 receives the electrical signal converted by the microphone 615 from the voice of the user operating the information processing terminal 22 as the output signal from the output signal transmission unit 91 of the information processing terminal 22. The communication unit 31 further receives an operation signal received by the information processing terminal 22 according to an operation performed by the user.

The user group determination unit 32 determines a group of two or more users on the basis of the position information and the directions of the users. The user group determination unit 32 determines two or more users facing each other or talking to each other, and determines that these users belong to a group.

The user guide unit 33 determines which of the image display devices 12 in the meeting room the user faces based on the position information and the direction of the user, and generates a work area of the user on the image display device 12. In the case where the image display devices 12 are projectors, the user guide unit 33 determines which of the image display devices 12 is projecting the screen that the user faces, and causes the determined image display device 12 to display a work area of the user. In other words, a user-dedicated work area is automatically prepared in a portion out of the entire screen of the image display device 12 in accordance with the position and the direction of the user.

The user information acquisition unit 34 acquires the activity information of the user received by the communication unit 31 from the sensor 14 and stores the activity information in time series for each user.

The generation unit 35 generates the sound data and the image data based on the behavior information of the multiple users in the meeting room as described later. The sound data and the image data generated by the generation unit 35 include data read from the storage unit 50.

The first environmental control unit 36 controls the output unit 92 of the information processing terminal 22 or the output unit 93 of the speaker 16 to output the tone (ambient sound) based on the sound data generated by the generation unit 35.

The second environmental control unit 37 controls the output unit 92 of the information processing terminal 22 or the image display device 12 to output the image or video based on the image data generated by the generation unit 35.

The psychological safety estimation unit 38 estimates or determines a psychological safety level based on the face-to-face time of the multiple users determined as a group. The psychological safety level is an example of the state of users, and the psychological safety estimation unit 38 serves as a user state determination unit. Alternatively, the psychological safety estimation unit 38 may estimate a psychological safety level according to the vital data of the multiple users determined as a group. Both the face-to-face time and the vital data may be used. Yet alternatively, the psychological safety estimation unit 38 may estimate a psychological safety level according to the speech data (e.g., voice volume) of the multiple users determined as a group. All the face-to-face time, the vital data, and the speech data may be used.

The minutes creation unit 39 creates minutes using the speech data of the users. The speech data (utterance) is constantly recorded as the activity information. The minutes are speech data recorded when two or more users are grouped.

The individuality evaluation unit 44 evaluates the diversity of individualities of users in a team to which the users belong. The team may be an organization team, such as a development team or a sales planning team, to which users are assigned; or a group to which users are grouped by some criterion.

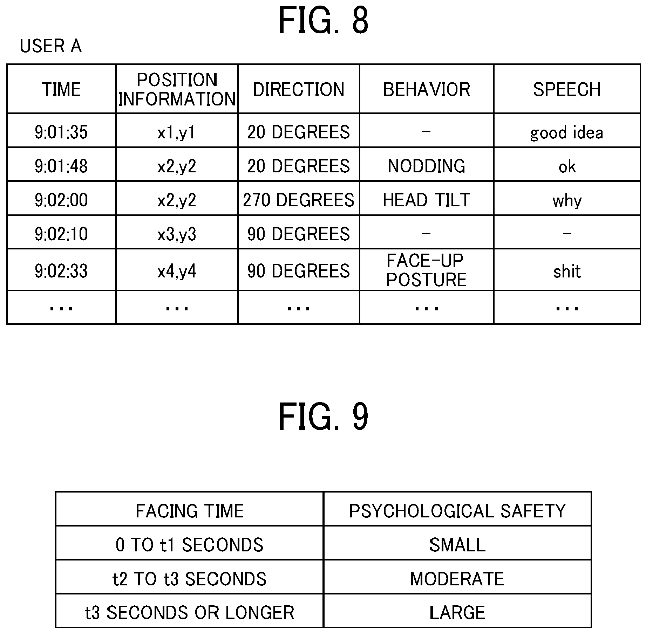

The storage unit 50 stores the activity information 51, the psychological safety information 52, and the tone and image information 53, for example, in a table format as illustrated in FIGS. 8 to 10. The activity information 51, the psychological safety information 52, and the tone and image information 53 are not necessarily in the table format as illustrated in FIGS. 8 to 10, and may be in any format in which similar information is stored.

For example, the individual relaxation level during an activity of a team is one index representing the level of psychological safety of the team. It is known in the art that the relaxation level can be estimated by measuring the balance of the autonomic nervous system from the fluctuations in heartbeat. The information processing system 10 measures the individual activity information by the activity sensors 9 and presents a report on the relaxation levels to the team. Further, the information processing system 10 outputs in real time, for example, a calm video or background music (BGM), or conversely an exciting video or BGM in accordance with the relaxation level.

FIG. 8 is a diagram illustrating a data structure of the activity information 51 according to the present embodiment. The activity information 51 in FIG. 8 includes items of time, position information, direction, behavior, and speech data.

The item of time is the Japan Standard Time on the day. The activity information may be recorded by day.

In the item of position information, the position (coordinates) of the user detected by communication between the UWB module 703 and the sensor 14 is stored.

In the item of direction, the direction in which the user faces is stored. Although the direction in FIG. 8 is indicated by the angle in the horizontal direction, the angle in the latitude direction (vertical direction) is also detected.

In the item of behavior, the behavior information representing the behavior of the user is stored.

In the item of speech, text data recognized from the sound data representing an utterance by the user is stored as speech data.

The position information and the direction are detected successively (for example, 10 times or more per second). By contrast, the behavior information and the speech data are event data detected only when a behavior and an utterance are detected, respectively. For this reason, in FIG. 8, the position information and the direction are illustrated in accordance with the event for the convenience of drawing, but the position information and the direction are successively detected at shorter intervals than the intervals illustrated in the drawing.

FIG. 9 is a diagram illustrating a data structure of the psychological safety information 52 according to the present embodiment. The psychological safety information 52 illustrated in FIG. 9 includes items of face-to-face time and level of psychological safety.

The item of face-to-face represents the time during which the user group determination unit 32 determines that the users are facing each other.

The psychological safety information 52 represents the level of psychological safety corresponding to face-to-face time. In other words, the level of psychological safety is estimated based on the knowledge that the longer the face-to-face time is, the higher the level of psychological safety is.

Although psychological safety is divided into three levels in FIG. 9, psychological safety may be divided into larger number of levels such as 10 levels of 1 to 10.

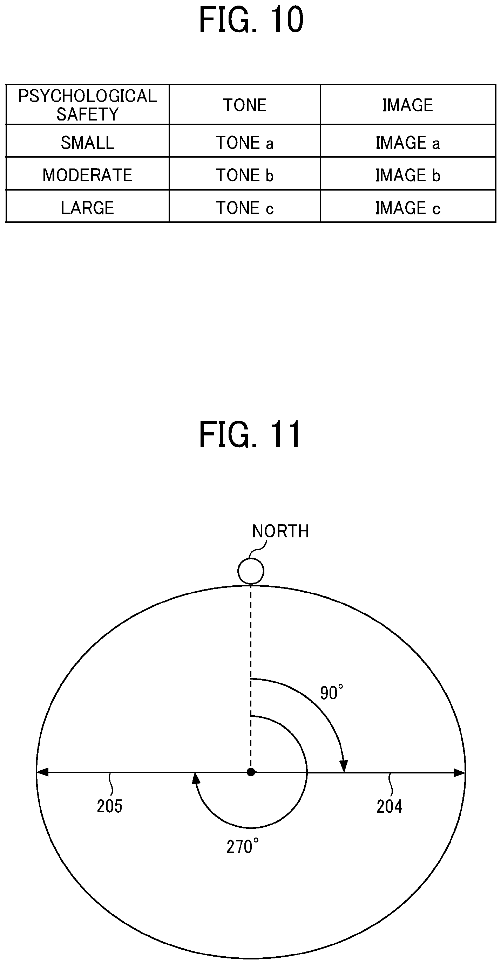

FIG. 10 is a diagram illustrating a data structure of the tone and image information 53 according to the present embodiment. The tone and image information 53 in FIG. 9 includes items of psychological safety, tone, and image.

The item of psychological safety is the same as or similar to that in FIG. 9.

The tone represents music associated with the level of psychological safety. For example, when the level of psychological safety is low, music for increasing the level of psychological safety is used, and when the level of psychological safety is high, music for obtaining more creative output is used. The tone is not limited to that stored in the tone and image information 53, but the information processing system 10 may download suitable music from the Internet. Further, the beats per minute (BPM) of, for example, music stored in the tone and image information 53 or acquired from the outside may be changed, the number of musical instruments may be increased, or the tone may be effectively changed.

The image represents an image or video associated with the level of psychological safety. For example, when the level of psychological safety is low, an image for increasing the level of psychological safety is displayed, and when the level of psychological safety is high, an image for obtaining a more creative output is displayed. The image is not limited to the images stored in the tone and image information 53, and the information processing system 10 may download images from the Internet. The image may be a still image or a moving image (video). Further, the image may be an illumination pattern in which illumination is brightened, darkened, or blinked; or a color scheme is rhythmically changed.

In FIG. 10, vital data such as heartbeat may be used instead of psychological safety. In this case, the first environmental control unit 36 outputs a tone corresponding to the vital data of the user, and the second environmental control unit 37 outputs an image corresponding to the vital data of the user.

In FIG. 10, the volume (loudness) of voice detected from speech data may be used instead of psychological safety. In this case, the first environmental control unit 36 outputs a tone corresponding to the voice volume of the user, and the second environmental control unit 37 outputs an image corresponding to the voice volume of the user.

### Determination of Group

The direction of the user will be described with reference to FIG. 11. FIG. 11 is a diagram illustrating the direction of the user according to the present embodiment. In FIG. 11, the direction is indicated as an angle in a clockwise direction from the north serving as a reference. In FIG. 1, a direction 204 is 90 degrees and a direction 205 is 270 degrees. When the direction reaches 360 degrees, the direction returns to 0 degree. The direction varies from 0 degree to 360 degrees. Although the horizontal direction is illustrated in FIG. 11, the direction of the user in the elevation direction is also detected.

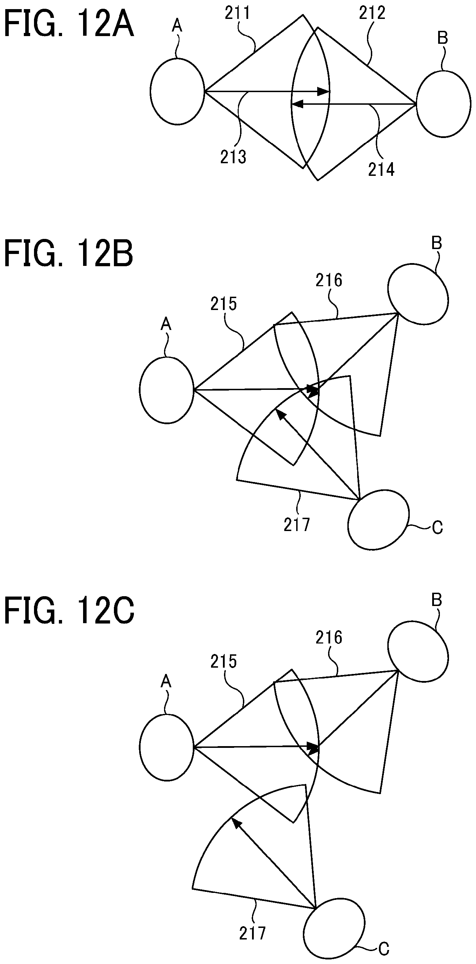

A description is given below of the grouping of users with reference to FIG. 12. FIGS. 12A, 12B, and 12C are diagrams each illustrating grouping of users according to the present embodiment. The term "group" refers to two or more users facing each other or having a conversation. The term "facing each other" refers to the situation in which the distance between the users is within a threshold distance, and their directions are directed toward each other. This situation may be referred to as a "face-to-face situation" in the following description.

FIG. 12A illustrates the two users A and B facing each other. As illustrated in FIG. 12A, when sectors 211 and 212 individually set for the users A and B overlap, the users face each other. The threshold distance for determining whether the users are facing each other is appropriately determined (for example, about 1 meter).

The user group determination unit 32 can determine whether two or more users facing each other or two or more users having a face-to face conversation correspond to close contact with an infected person in a manner similar to the manner of determination of group. The term "close contact" refers to, for example, a person who touches an infected person with his/her hand without taking necessary measures to prevent infection, or a person who stays 15 minutes or longer within such a distance (about 1 m) that his/her hand touches a hand of the infected person if their hands are extended.

The center of the sector 211 is a direction 213, and the center of the sector 212 is a direction 214. The angle that defines the spread of the sectors 211 and 212 is determined in advance. The angle defining the spread is set to such an angle that the users are presumed to be facing each other. A description is given of determining whether the users face each other according to the present embodiment, assuming that the angle defining the spread is 30 degrees in the forward and backward directions with respect to the directions 213 and 214. When the direction 213 is at 90 degrees, the direction opposite thereto (different by 180 degrees) is at 270 degrees by adding 90 degrees and 180 degrees. Accordingly, when the direction 214 is within a range of 240 (= 270 - 30) degrees to 300 (= 270 + 30) degrees, the user group determination unit 32 determines that the user A faces the user B.

FIG. 12B illustrates the three users A to C facing each other. The method of determining whether three or more users face each other is similar to the method described above. When sectors 215 to 217 individually set for the users A to C overlap, the users face each other. The sector 215 of the user A overlaps the sector 216 of the user B, the sector 215 of the user A overlaps the sector 217 of the user C, and the sector 216 of the user B overlaps the sector 217 of the user C. Accordingly, the users A to C face each other.

Although the case of three users is described in FIG. 12B, a similar method may be applied to the case of four or more users. The distance between the users increases as the number of users having a conversation increases. Accordingly, the threshold distance between the users may also increase according to the number of users.

It is not necessary to determine that the sectors of the three users overlap each other. For example, in the case illustrated in FIG. 12C, the sector 215 of the user A overlaps the sector 216 of the user B, and the sector 215 of the user A overlaps the sector 217 of the user C, but the sector 216 of the user B does not overlap the sector 217 of the user C. In this case, since the user A faces the user B and the user A faces the user C, the user group determination unit 32 may determine that the user B faces the user C.

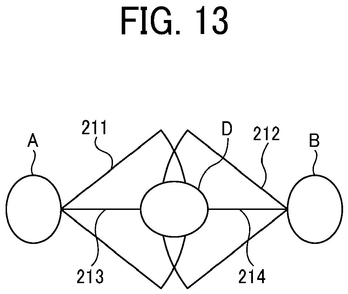

Further, as illustrated in FIG. 13, when another user D is present between the two users A and B, the user group determination unit 32 does not determine that the user A faces the user B. FIG. 13 is a diagram illustrating a method of determining whether users face each other when another user is present according to the present embodiment. In this case, based on the direction of a user D, the user group determination unit 32 determines that the users A and D or the users A and B face each other.

### Output of Tone or Image corresponding to Psychological Safety Level

A supplemental description is given of psychological safety levels. Psychological safety refers to an environment having a gentle atmosphere in which members are encouraged to act naturally without being afraid of the reactions of others or feeling embarrassed. The psychological safety level is its degree. The gentle atmosphere refers to an atmosphere in which members can naturally convey his/her thoughts and feeling and creative output is expected.

In the present embodiment, the psychological safety estimation unit 38 estimates the psychological safety level based on, for example, the time during which the users face each other, the vital data such as heartbeat, or the voice volume. As a result, the information processing system 10 can output a tone or an image corresponding to the psychological safety level, to promote creative output.

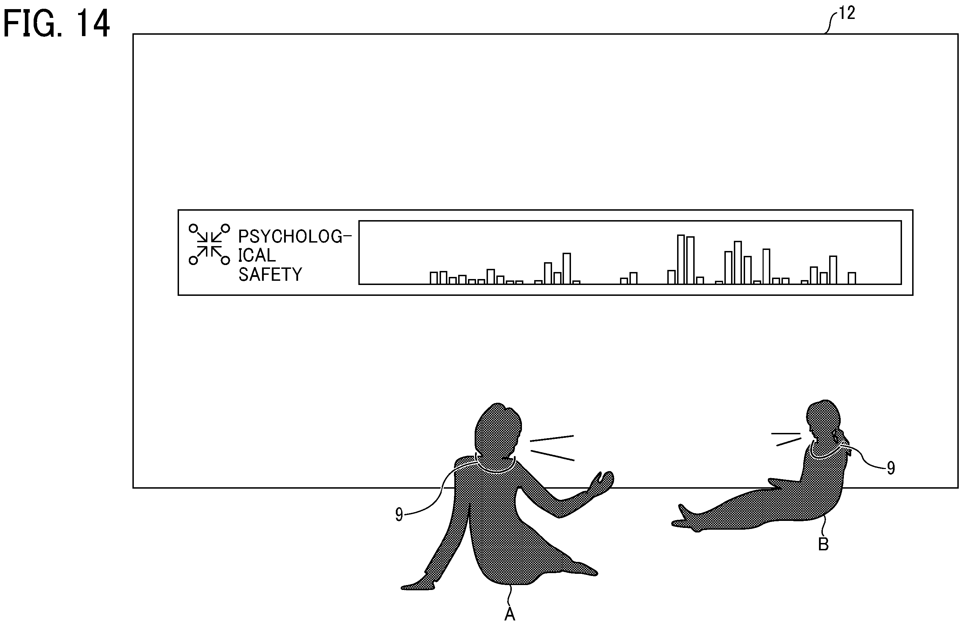

FIG. 14 illustrates the two users A and B facing each other. For example, in a case where users A and B who are a superior and a subordinate face each other, if the psychological safety level is low, the information processing system 10 outputs a tone or an image that enhances the psychological safety level.

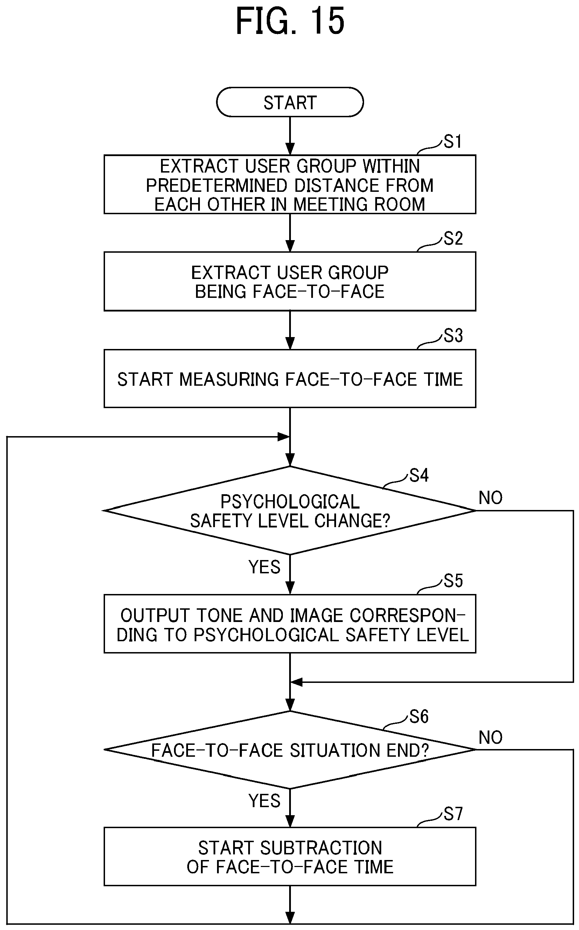

FIG. 15 is a flowchart of a process of outputting a tone and an image corresponding to the psychological safety level, performed by the information processing system 10 according to the present embodiment. The process of FIG. 15 is executed for a user whose activity information is detected.

The user information acquisition unit 34 acquires the activity information of the user repeatedly transmitted from the sensor 14 via the communication unit 31, and stores the activity information in time series on an individual user basis. The same applies to the flowchart of FIG. 15 and flowcharts of the subsequent drawings. The user group determination unit 32 extracts a group of users within a threshold distance from each other in the meeting room (S1). By limiting the range to the inside of the meeting room, the processing load of the information processing system 10 can be reduced. The presence in the meeting room is determined as the sensor 14 dedicated to the individual meeting room receives the activity information. The process of FIG. 15 is applicable to not only the inside of the meeting room but also other locations such as a corridor.

Subsequently, the user group determination unit 32 extracts a group of users whose directions are directed toward each other among the users within the threshold distance from each other (S2).

The user group determination unit 32 starts measuring the face-to-face time of the group of users extracted in step S2 (S3).

The psychological safety estimation unit 38 acquires the psychological safety level corresponding to the time during which the users face each other, the vital data, or the speech data from the psychological safety information 52, and determines whether the estimated psychological safety level has changed (S4).

When the determination in step S4 is Yes, the first environmental control unit 36 outputs a tone corresponding to the psychological safety level, and the second environmental control unit 37 outputs an image corresponding to the psychological safety level (S5). When the determination in step S4 is No, the process proceeds to step S6.

The user group determination unit 32 determines whether the face-to-face time ends based on the positions and the directions of the users (S6).

In a case where the determination in step S6 is Yes, the user group determination unit 32 starts subtraction of the face-to-face time (S7). Gradually reducing the face-to-face time from the end of the face-to-face situation in this manner is advantageous. When the users again face each other, the user group determination unit 32 can resume measuring time from the face-to-face time being subtracted. When the determination in step S6 is No, the process proceeds to step S4 while the measurement of the face-to-face time is continued.

In this way, the information processing system 10 can output a tone or an image corresponding to the psychological safety level, and can promote creative output from the users facing each other.

In FIG. 15, whether the users are having a conversation is not considered in determining whether the users face each other. The user group determination unit 32, however, may determine that the users face each other only when the users are having a conversation.

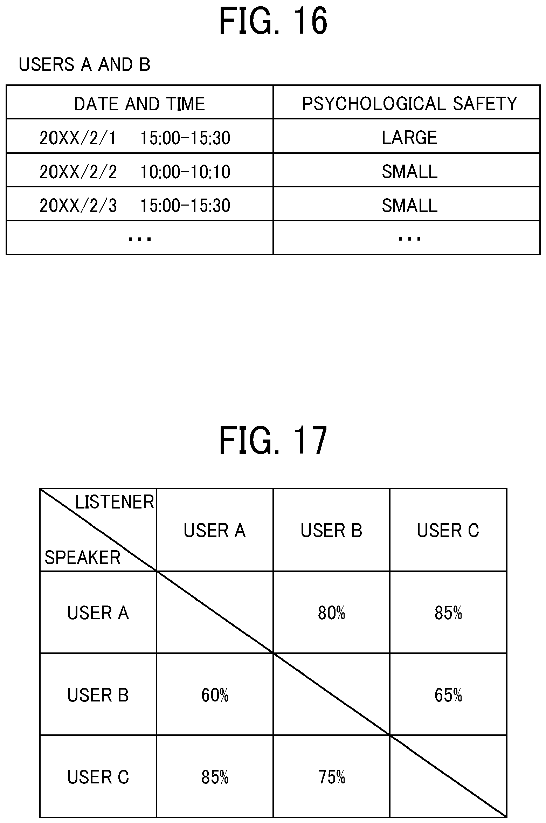

As illustrated in FIG. 16, it is also effective to record the transition of the psychological safety level between users. FIG. 16 is a diagram illustrating how the psychological safety level between the users A and B changes day by day. The user group determination unit 32 records the highest psychological safety level in association with the time, for example, on a daily basis.

In FIG. 16, the psychological safety level between the users A and B decreases with the day. When such information is stored in the information processing system 10, for example, the manager of the organization to which the users belong can determine whether it is necessary to repair the relationship between the users A and B. Further, the information processing system 10 can output a tone or an image preferable for the relationship between the users A and B.

Further, multiple pairs of users facing each other may be in one meeting room. For example, the users A and B face each other, and the users C and D face each other. In this case, the psychological safety estimation unit 38 may calculate, as the psychological safety level, the average of the psychological safety level of the users A and B and the psychological safety level of the users C and D. Alternatively, the first environmental control unit 36 may use acoustic technologies to output tones individually corresponding to the psychological safety levels of the user pairs. In other words, the first environmental control unit 36 outputs different tones to the pair of users A and B and the pair of users C and D.

Similarly, the second environmental control unit 37 may output an image corresponding to the psychological safety level of the pair of users A and B to a wall (or a part of the wall) close thereto and an image corresponding to the psychological safety level of the pair of users C and D to a wall (or a part of the wall) close thereto.

### Scoring Likelihood of Occurrence of Psychological Safety

The likelihood of occurrence of psychological safety may be scored with respect to the activity in the team of users. It is known in the art that behaviors in communication contribute to, for example, human relationships, psychological safety, and reliability, which are necessary for team creativity (quality for producing creative results). In particular, for example, a human relationship appears in how a listener listens to a speaking person. Nodding and chiming in are typical examples of the manner of active listening. Based on such knowledge, the psychological safety estimation unit 38 detects the directions and nods (or reactions) of the users from the activity information, to measure the level of active listening of listeners around a certain speaker. The psychological safety estimation unit 38 collects and analyzes the active listening information on a long-term basis, periodically returns a report of the collected information to the team, and provides the team with materials for team building. The term "team building" used in this disclosure refers to organizing teams by considering to which team the user is to be assigned. Using the active listening information, for example, when the level of active listening to a certain utterance is low, the environmental control system 100 can perform the environmental control to increase the concentration of surrounding listeners. For example, the first environmental control unit 36 reduces the volume of BGM, and the second environmental control unit 37 increases the illuminance of the image or the lighting.

Note that whether certain users belong to the same team may be determined using team information registered in advance, for example, in human resources information and indicates members belonging to the team. Alternatively, users who have been grouped a threshold number of times or more in the past may be regarded as a team.

FIG. 17 is a table presenting the calculated percentages of facing time (i.e., time during which the listener faces the speaker) in the team according to the present embodiment. The percentage of time used here refers to the proportion of the time during which the listener faces the speaker relative to the time of conversation between the speaker and the listener grouped. In FIG. 17, the row heading indicates the users A to C as speakers, and the column heading indicates the users A to C as listeners. For example, when the user A is the speaker, the percentage of time during which the user B faces the user A is 80%, and the percentage of time during which the user C faces the user A is 85%.

The psychological safety estimation unit 38 calculates the average value and the standard deviation of all the percentages in the team. It is preferable for the team to have a high average value. However, when the standard deviation is large, the level of active listening differs among the team members, and it is presumed that the psychological safety level is unlikely to occur even if the average value is high. The psychological safety estimation unit 38 scores the standard deviation of frequencies of nodding in FIG. 17 in a range of, for example, 0 to 100, or weights the average value and the standard deviation to score the likelihood of occurrence of psychological safety of the team in a range of, for example, 0 to 100.

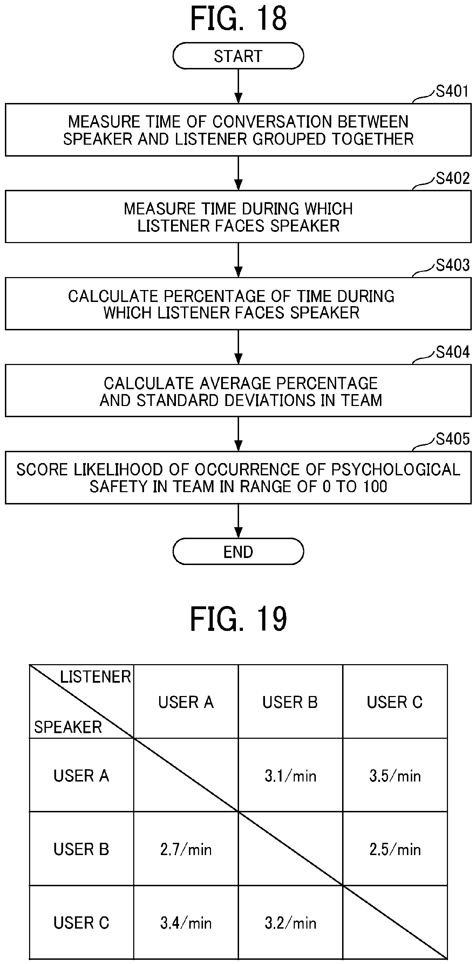

FIG. 18 is a flowchart of a process of scoring the likelihood of occurrence of psychological safety of a team based on the percentages of time during which the listener faces the speaker, performed by the psychological safety estimation unit 38 according to the present embodiment.

The psychological safety estimation unit 38 measures the time of a conversation between the speaker and the listener grouped (S401). Alternatively, simply the time during which multiple users are grouped may be measured regardless of whether the users are talking.

The psychological safety estimation unit 38 measures the time during which the listener faces the speaker (S402).

The psychological safety estimation unit 38 calculates the percentage of the time during which the listener faces the speaker (S403).

The psychological safety estimation unit 38 calculates the average value and the standard deviation of the percentages of all the listeners in the team (S404). The average value and the standard deviation of the percentages of all the listeners in the team may be displayed on the information processing terminal 22 or may be output by voice.

The psychological safety estimation unit 38 scores the likelihood of occurrence of psychological safety of the team in a range of, for example, 0 to 100 (S405). After the scoring, in a case where at least a part of the team members starts a conversation again and the score of the team is equal to or lower than a threshold, the environmental control system 100 can perform the environmental control to increase the concentration of surrounding listeners. For example, the first environmental control unit 36 reduces the volume of BGM, and the second environmental control unit 37 increases the illuminance of the image or the lighting.

FIG. 19 is a table presenting the calculated frequencies of nodding of the listeners to the speaker in the team according to the present embodiment. In FIG. 17, the row heading indicates the users A to C as speakers, and the column heading indicates the users A to C as listeners. The behavior information of nodding is included in the activity information. The frequency of nodding in FIG. 19 is calculated as follows. The number of times of nodding by the listener during the time of conversation between the speaker and the listener grouped is converted into the number of times of nodding per unit time (for example, 1 minute). For example, when the user A is the speaker, the frequency of nodding of the user B is 3.1 times and the frequency of nodding of the user C is 3.5 times.

The psychological safety estimation unit 38 calculates the average value and the standard deviation of all the frequencies of nodding in the team. It is preferable for the team to have a high average value. However, when the standard deviation is large, the level of active listening differs among the team members, and it is presumed that the psychological safety level is unlikely to occur even if the average value is high. Since whether a person nods varies greatly from person to person, it is preferable that a certain upper limit be used for frequencies of nodding equal to or greater than a threshold. Such an upper limit can prevent an increase in the standard deviation in a case where psychological safety is likely to occur (human relationship is good). The psychological safety estimation unit 38 scores the standard deviation of frequencies of nodding in FIG. 19 in a range of, for example, 0 to 100, or weights the average value and the standard deviation to score the likelihood of occurrence of psychological safety of the team in a range of, for example, 0 to 100.

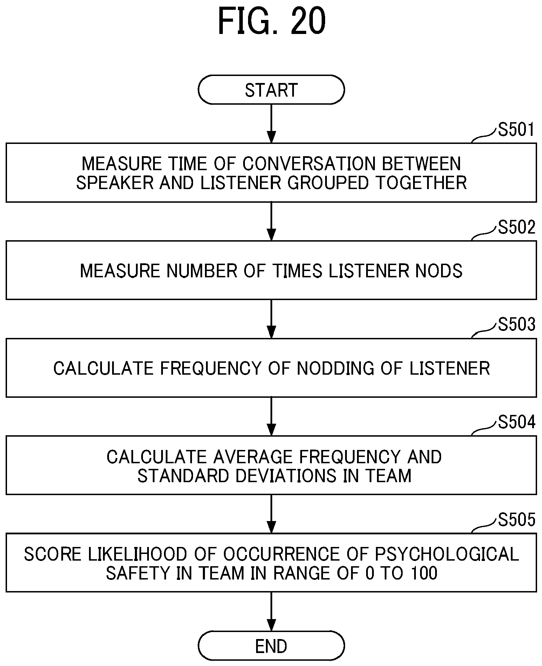

FIG. 20 is a flowchart of a process of scoring the likelihood of occurrence of psychological safety of a team based on the frequencies of nodding of a listener to a speaker, performed by the psychological safety estimation unit 38 according to the present embodiment.

The psychological safety estimation unit 38 measures the time of a conversation between the speaker and the listener grouped (S501). Alternatively, simply the time during which multiple users are grouped may be measured regardless of whether the users are talking.

The psychological safety estimation unit 38 measures the number of times of nodding of the listener in the time measured in step S501 (S502).

From the time measured in step S501 and the number of times measured in S502, the psychological safety estimation unit 38 calculates the frequency of nodding of the listener in a unit time (S503).

The psychological safety estimation unit 38 calculates the average value and the standard deviation of the frequencies of all the listeners in the team (S504). The average value and the standard deviation of the frequencies of all the listeners in the team may be displayed on the information processing terminal 22 or may be output by voice.

The psychological safety estimation unit 38 scores the likelihood of occurrence of psychological safety of the team in a range of, for example, 0 to 100 (S505). After the scoring, in a case where at least a part of the team members starts a conversation again and the score of the team is equal to or lower than a threshold, the environmental control system 100 can perform the environmental control to increase the concentration of surrounding listeners. For example, the first environmental control unit 36 reduces the volume of BGM, and the second environmental control unit 37 increases the illuminance of the image or the lighting.

The psychological safety estimation unit 38 may weight the average value and the standard deviation of the percentages in FIG. 17 and the average value and the standard deviation of the frequencies of nodding in FIG. 19, to score the likelihood of occurrence of psychological safety of the team in a range of, for example, 0 to 100. The psychological safety estimation unit 38 periodically feeds back to the team members the score of the team as a report. Further, the psychological safety estimation unit 38 may display the score of the team when a team member approaches the image display device 12.

### Creation of Minutes in which Conversation Partner is Identified

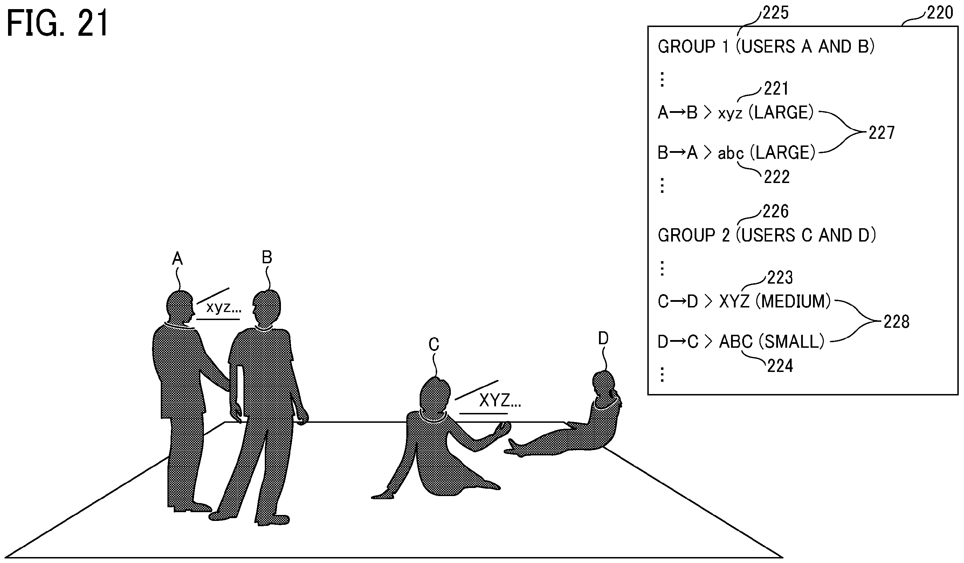

A description is given below of the creation of minutes of conversations among grouped users, with reference to, for example, FIG. 21. As illustrated in FIG. 21, by grouping the users, the minutes creation unit 39 can create the minutes 220 in which the conversation partner is identified. FIG. 21 is a diagram illustrating the creation of minutes 220 in which the conversation partner is identified, according to the present embodiment.

In FIG. 21, the users A and B have a face-to-face conversation, and the users C and D have a face-to-face conversation. In this case, the minutes creation unit 39 records, in the minutes 220, that speech data 221 represents an utterance of the user A to the user B, speech data 222 represents an utterance of the user B to the user A, speech data 223 represents an utterance of the user C to the user D, and speech data 224 represents an utterance of the user D to the user C, together with the speech data 221, 222, 223, and 224. In other words, the speech data is recorded together with the information identifying which of the multiple users grouped is a speaker and which of the multiple users is a listener.

When three or more users are grouped, the minutes creation unit 39 may determine that the conversation is performed between the users whose directions are directed most toward each other. This is because even in a group of three or more users, a speaker (speech source) often directs his/her body toward listeners (speech destinations).

When three or more users are grouped, the minutes creation unit 39 preferably records group identification information identifying the groups 225 and 226 to which the grouped users belong, respectively. Such a manner of recording facilitates extracting only the speech data of the users who uttered words in the same group from the minutes when the minutes are referred to later.

Further, the minutes creation unit 39 preferably records current psychological safety levels 227 and 228 in speech data, respectively. Such a manner of recording facilitates extracting only the speech data having psychological safety levels equal to or higher (or equal to or lower) than a threshold from the minutes when the minutes are referred to later. How much the psychological safety level affects the utterance can be analyzed, for example, by the manager of the organization.

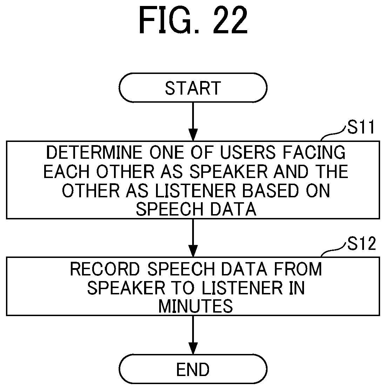

FIG. 22 is a flowchart of a process of creating minutes in which the conversation partner is identified, according to the present embodiment.

In the process of FIG. 22, it is assumed that the users facing each other are already identified and the grouping is completed.

The minutes creation unit 39 determines that one of the users facing each other is the speaker and the other is the listener based on the speech data (S 11). In other words, of the two users facing each other, the user who utters words is the speaker (speech source), and the other is the listener (listener).

Then, the minutes creation unit 39 records, in the minutes 220, the speech data representing the utterance from the speaker to the listener (S 12). The minutes creation unit 39 records the current psychological safety level in the minutes 220 in association with the speech data.

### Guidance Based on User Position and Direction

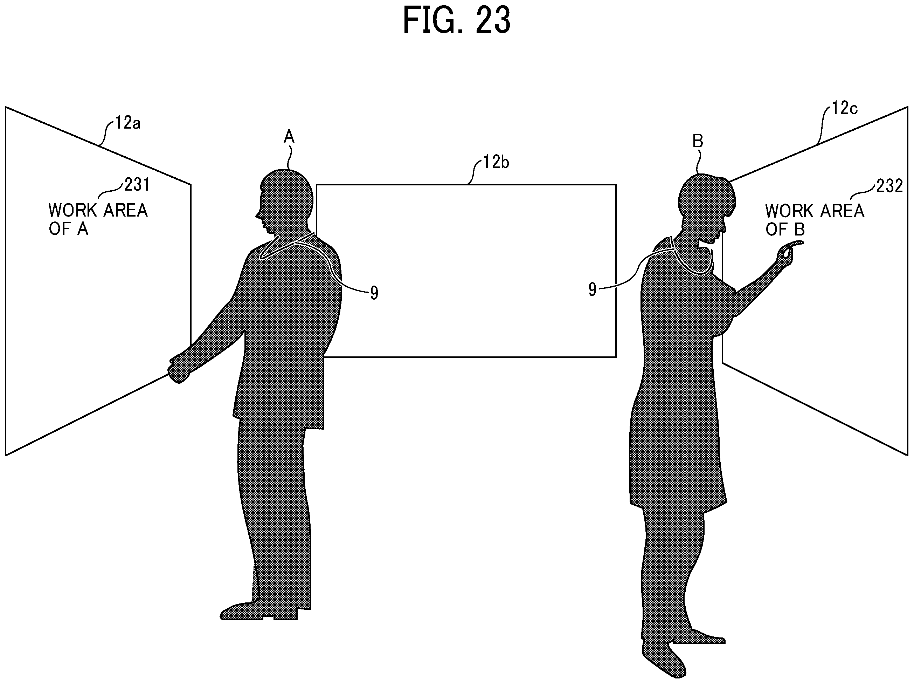

A description is given below of guidance to an appropriate image display device 12 based on the position and the direction of the user. When there are multiple image display devices 12 in the meeting room, the user guide unit 33 determines the image display device 12 (or a partial region of the image display device 12) to be used by the user according to which image display device 12 the user faces, as described below with references to FIG. 23.

FIG. 23 is a diagram illustrating the image display devices 12 to which the users face. FIG. 23 illustrates the three image display devices 12a to 12c. Since each of the image display devices 12a to 12c is a projection surface of a projector or a large touch panel, a user can use any of the image display devices 12a to 12c as a display device or an input device.

When the user A is within a threshold distance from the image display device 12a and faces the image display device 12a, the user guide unit 33 determines that the image display device 12a is to be used by the user A and guides the user A to the image display device 12a. For example, the user guide unit 33 displays a message 231 "work area of the user A" on the image display device 12a. When the user B is within the threshold distance from the image display device 12c and faces the image display device 12c, the user guide unit 33 determines that the image display device 12c is to be used by the user B and guides the user B to the image display device 12c. For example, the user guide unit 33 displays a message 232 "work area of the user B" on the image display device 12c. In the messages, "A" and "B" are, for example, names, identifiers (IDs), or nicknames of the users. In the information processing system 10, the name, the ID, or the nickname of the user is associated with the user ID set in the activity sensor 9. The information processing system 10 identifies, for example, the name associated with the user ID transmitted from the activity sensor 9.

The messages 231 and 232 are erased in response to a user operation or the elapse of certain time, and a work area dedicated to the user, which will be described later, is displayed. As described above, when the user approaches the image display device 12, the user can use the whole or a part of the image display device 12 as his/her work area.

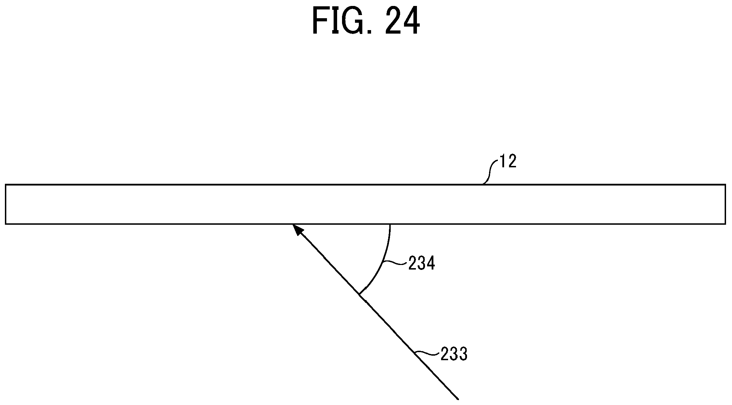

With reference to FIG. 24, a description is given of a method of determining whether the user is facing the image display device 12, according to the present embodiment. FIG. 24 is a diagram illustrating the relation between a direction 233 of the user and the surface of the image display device 12 according to the present embodiment. The user guide unit 33 calculates an acute angle 234 between the direction 233 of the user and the surface of the image display device 12. When the acute angle 234 is equal to or greater than a threshold (for example, 70 degrees), the user guide unit 33 determines that the user is facing the image display device 12.

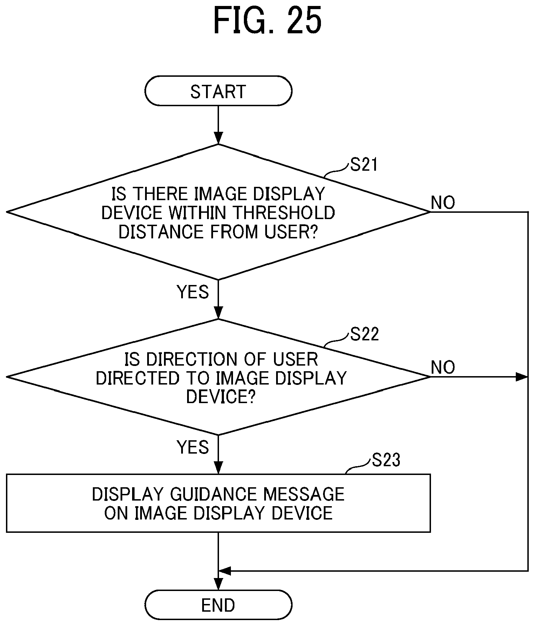

FIG. 25 is a flowchart of a process of guiding a user to the image display device 12, performed by the user guide unit 33 according to the present embodiment.

For example, every time the position of the user is detected, the user guide unit 33 determines whether there is the image display device 12 within the threshold distance from the user (S21). The distance between the user and the image display device 12 may be the shortest distance.

If the determination in step S21 is Yes, the user guide unit 33 determines whether the direction of the user is directed to the image display device 12 (S22). When the result of the determination of S21 is No, the process of FIG. 25 ends.

If the determination in step S22 is Yes, the second environmental control unit 37 displays a guidance message on the image display device 12 which the user faces (S23). When the result of the determination of S22 is No, the process of FIG. 25 ends.

After displaying the message, the user guide unit 33 erases the message in response to a user operation or the elapse of a certain time, and displays a work area dedicated to the user. When the user guide unit 33 determines that the user has confirmed the message based on the face direction of the user, the second environmental control unit 37 may erase the message and display the work area.

When the user guide unit 33 guides the user, the second environmental control unit 37 controls the image display device 12 to display a work area for the user and displays the user's speech data on the work area. In other words, the image display device 12 displays data input by voice or handwriting (or hand drafting). Handwritten data may be subjected to speech recognition in real time. The user can operate a Web browser in the work area to display a Web page or a desktop screen of his/her PC.

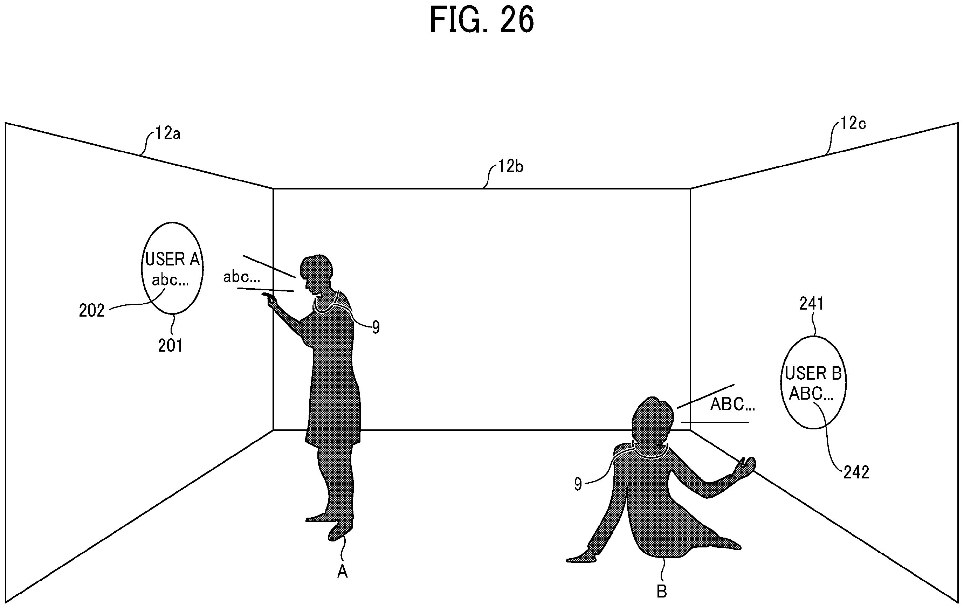

FIG. 26 is a diagram illustrating voice input to the image display devices 12 by the users, according to the present embodiment. The second environmental control unit 37 displays the speech data 202 input by voice by the user A in the work area 201 of the image display device 12a to which the user A has been guided. The second environmental control unit 37 displays speech data 242 input by voice by the user B in a work area 241 of the image display device 12c to which the user B has been guided. The work area is a dedicated display input area that can be freely used by the user. Information input to the work area by the user is recorded in association with the user.

The work areas 201 and 241 each have a fixed shape and size in the initial state. The shape does not need to be circular as illustrated in the figure, but may be, for example, rectangular or polygonal. When the amount of speech data input by the user increases, the second environmental control unit 37 automatically widens the work areas 201 and 241. When the speech data further increases, the second environmental control unit 37 may display a scroll bar. The second environmental control unit 37 may change the shape or size of the work areas 201 and 241 according to a user operation. The second environmental control unit 37 may move the work areas 201 and 241 as desired according to dragging or a voice input by the user.

When the user guide unit 33 allocates, as work areas, areas of one image display device 12 to multiple users, the second environmental control unit 37 adjusts the positions, sizes, and shapes thereof so that the multiple work areas do not overlap.

Displaying the work areas 201 and 241 in this manner are applicable to the following scenes.
(i) The users individually concentrate on thinking up their ideas.

In this case, the speech data of the users A and B are sequentially displayed in the work areas 201 and 241.
(ii) One user presents his/her thoughts to another user.

In this case, the user can explain the content of the speech data with the speech data of the users A and B respectively displayed in the work areas 201 and 241.
(iii) Multiple users have a meeting (discussion).

In this case, the speech data of the users are displayed in their respective work areas.

The users can move to the work area of another user or copy and paste the speech data from and to the work area of another user, to gather ideas. Note that the second environmental control unit 37 displays their respective work areas at the positions close to the users so as not to overlap. When multiple users are grouped, the minutes are also recorded.

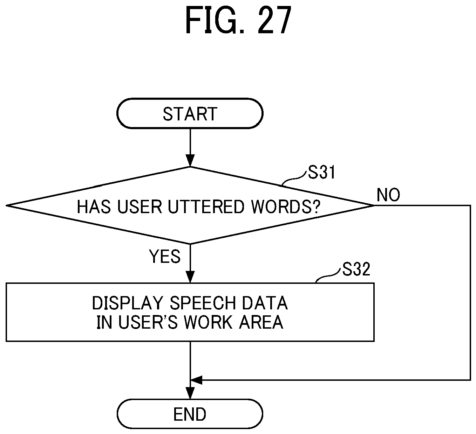

FIG. 27 is a flowchart of a process of displaying user's speech data in a work area, performed by the second environmental control unit 37 according to the present embodiment.

The second environmental control unit 37 determines whether the user has uttered words (S31). Which user has uttered words is identified by the user ID transmitted by the activity sensor 9. The user's utterance is constantly recorded.

When the user utters words (Yes in S31), the second environmental control unit 37 displays the speech data in his/her work area (S32).

In this way, the second environmental control unit 37 displays the speech data on the image display device 12 near the user who has uttered words. The user can input ideas by voice or handwriting at any time. When the user group determination unit 32 determines that multiple users face each other near the image display device 12, the users can have a discussion using the image display device 12.

The work area can be displayed on, not limited to a wall, but also a floor or a ceiling provided with the image display device 12.

### Team Individuality Evaluation Based on Utterance Content

It is known in the art that individuality is reflected in utterance contents. The exposure and diversity of individualities are desirable in the creativity of a team such as a development team. A description will be given of a method in which the individuality evaluation unit 44 digitizes variations in the individuality of team members from the utterance contents (keywords).

The individuality evaluation unit 44 determines, for example, favorite fields, fields of interest, and specialty fields of the individual team members based on the keywords extracted from utterance contents. The individuality evaluation unit 44 may change the weight of the keyword in accordance with the volume or speed of utterance of the keyword. For example, the weight is larger as the voice is louder or the speed is higher. The individuality evaluation unit 44 infers the level of expression of individuality and the diversity of individuality of the team, using the variations (standard deviation) of the determined fields among the team members.

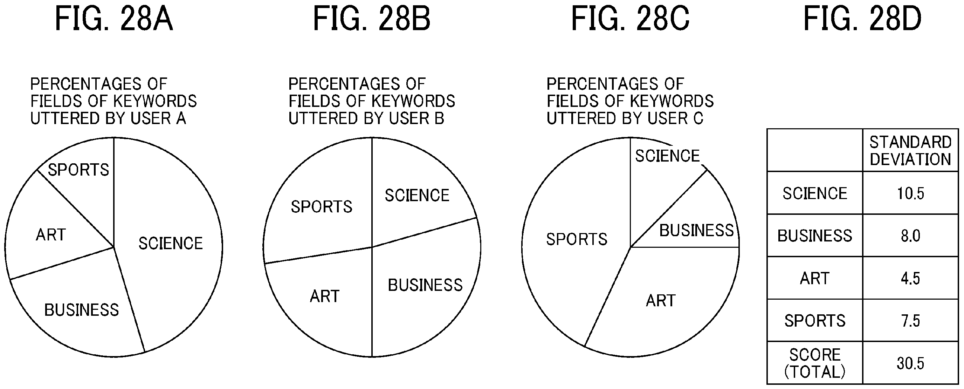

A description is given of the evaluation of individuality based on a keyword according to the present embodiment, with reference to FIGS. 28A to 28D. FIGS. 28A to 28D are graphs each illustrating a method of evaluating individuality based on speech data according to the present embodiment. FIG. 28A illustrates the percentages of fields to which the keywords extracted from the speech data of the user A belong. Specifically, the individuality evaluation unit 44 performs morphological analysis on the speech data to extract, for example, nouns and determines the field to which the nouns belong. Examples of fields include science, business, art, and sports. In determining the field, a dictionary in which keywords and fields are associated with each other in advance or classification based on artificial intelligence (AI) may be used. FIG. 28A illustrates the percentage of the number of keywords belonging to a field relative to the number of all keywords extracted from the speech data of the user A.

Similarly, FIG. 28B illustrates the percentages of fields to which the keywords extracted from the speech data of the user B belong. FIG. 28C illustrates the percentages of fields to which the keywords extracted from the speech data of the user C belong. The percentage is calculated based on the speech data for a certain period such as the past one month, half a year, or one year.

When the graphs of the user A, the user B, and the user C are compared with each other, it can be seen that the percentage of the field of science is greatly different, but the percentage of the field of art is similar. In order to quantify such differences, the individuality evaluation unit 44 calculates, for each field, a standard deviation of the percentages among the speakers. A large standard deviation means that the individuality of the team is diverse.

FIG. 28D illustrates the standard deviations of the percentages of fields and the sum thereof. Specifically, the standard deviation of science is 10.5, which is the largest, and the standard deviation of art is 4.5, which is the smallest. It can be determined that the larger the sum of the standard deviations is, the more diverse the individuality of the team becomes. Accordingly, the individuality evaluation unit 44 uses the sum or average of the standard deviations of all the fields as the variation score of the individuality of the team.

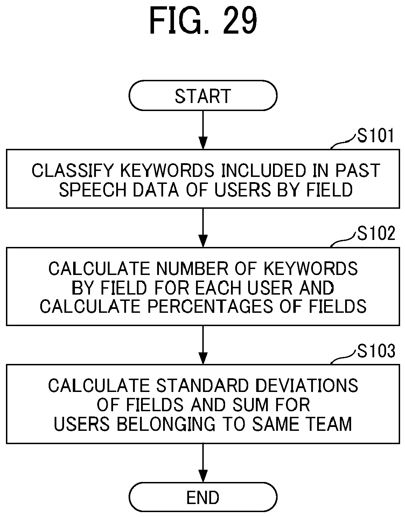

FIG. 29 is a flowchart of a process of scoring the individuality of a team, performed by the individuality evaluation unit 44 according to the present embodiment. The process of FIG. 29 may be performed, for example, at regular intervals or in response to a request from a user or the manager of the organization to the information processing system 10 via the information processing terminal 22.

The individuality evaluation unit 44 extracts the speech data of the individual users in a certain past period from the activity information, and classifies keywords included in the speech data by field (S101).

The individuality evaluation unit 44 calculates, for each user, the number of keywords by field, and calculates the percentage of the number of keywords by field relative to the total number of keywords (S 102). At this time, the individuality evaluation unit 44 may increase the number of keywords in accordance with the volume or speed of utterance of the keyword, and increase the total number by the same number.

The individuality evaluation unit 44 calculates, for each field, a standard deviation among members belonging to the same team, and calculates the total or average of the standard deviations of the fields as a variation score (S 103). The individuality evaluation unit 44 may display the score of the team when a team member approaches the image display device 12, or may output the score to team members or the human resources department by, for example, email.

In this way, the individuality evaluation unit 44 determines whether the individualities of members belonging to the same team are diversified.

The environmental control system 100 of the present embodiment can control the environment of the meeting room by using the position information and the direction information of the user or the position information, the direction information, and the speech data of the user.

### Embodiment 2

In the present embodiment, environmental control using user behavior information will be described.

### Functions

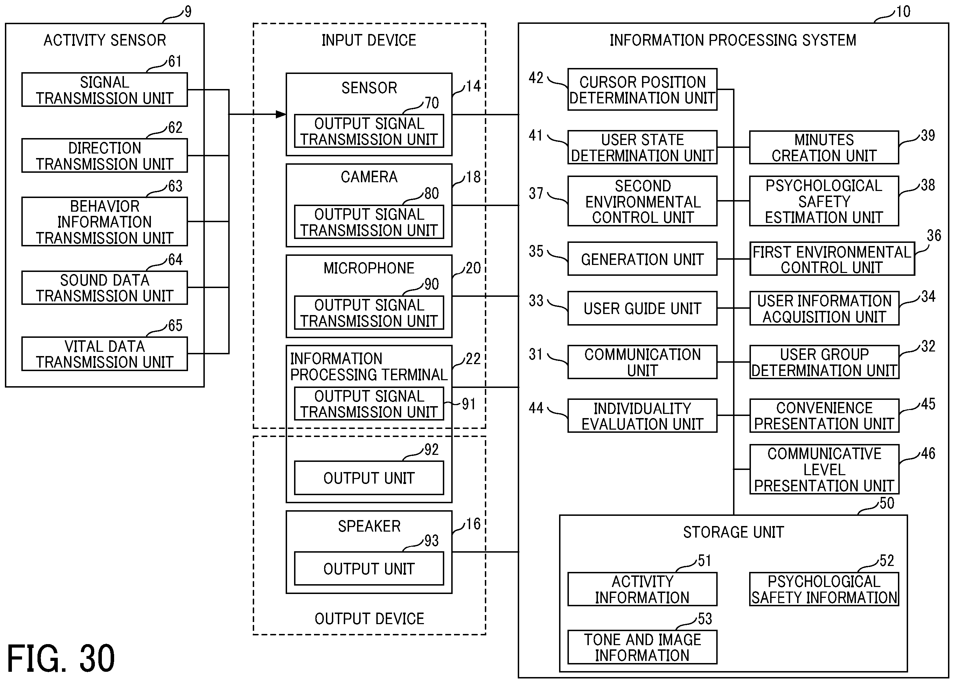

FIG. 30 is a block diagram illustrating a functional configuration of the information processing system 10 according to the present embodiment. Like reference signs are given to elements in FIG. 30 similar to those illustrated in FIG. 7 as those elements operate similarly and provide the same or similar effects, and redundant descriptions may be omitted in the following description.

The information processing system 10 according to the present embodiment includes a user state determination unit 41, a cursor position determination unit 42, a convenience presentation unit 45, and a communicative level presentation unit 46. The user state determination unit 41 determines, for example, the understanding level and concentration level of the user in a conversation in consideration of the behavior information.

The cursor position determination unit 42 determines the cursor position on the image display device 12 based on the direction of the user. The cursor is a small shape or symbol (for example, a mouse pointer) indicating the current input position on the operation screen of a computer.

The convenience presentation unit 45 evaluates the convenience of a place in an office and enables optimization of the layout of the office. The convenience evaluated by the convenience presentation unit 45 is the quantification, for each place in an office, of the usage rate, the concentration levels of users, the psychological safety levels (relaxation levels), and the conversation volumes.

The communicative level presentation unit 46 presents a place where meaningful communications are performed in the office. In a place where certain persons have meaningful communication, it is easy for other users to have meaningful communications. Accordingly, the user can select such a place to have communication.

### User State Determination

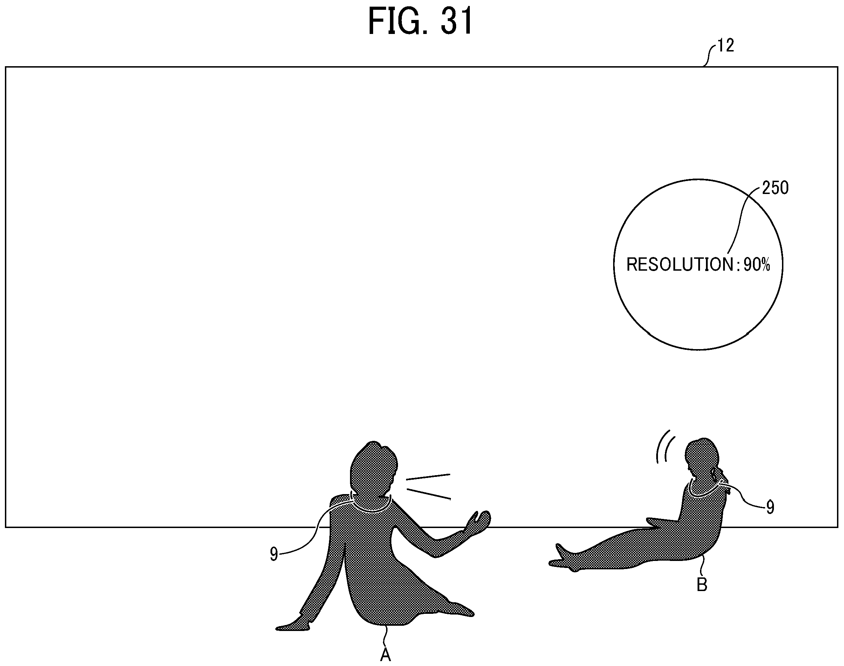

A description is given of a method of determining a user state using the behavior information with reference to FIG. 31. FIG. 31 is a diagram illustrating a method of determining an understanding level as a user state. In FIG. 31, the users A and B have a face-to-face conversation. The user state determination unit 41 detects a nod or a head tilt of the listener while another person is speaking or at the end of speaking, so as to determine the understanding level of the listener. The second environmental control unit 37 displays an understanding level 250 on the image display device 12 in real time.

For example, the user state determination unit 41 counts the number of times of nodding and head tilt of the users facing each other. The user state determination unit 41 deducts the number of times of head tilt from the number of times of nodding in a certain past time and converts the calculated value into the understanding level 250. For example, the understanding level 250 is determined in proportion to the value obtained by deducting the number of times of head tilt from the number of times of nodding. The understanding level 250 may be a numerical value such as 0 to 100% or may be, for example three levels of large, medium, and small.

The user state determination unit 41 may determine the understanding level by using speech data. For example, in response to detecting utterances such as "I see" and "uh-huh," the user state determination unit 41 determines that the understanding level has enhanced. The user state determination unit 41 may determine the understanding level from the speech data by using a model obtained by machine learning of the correspondence between speech data and understanding levels.

Preferably, the first environmental control unit 36 outputs a tone corresponding to the understanding level of the user, and the second environmental control unit 37 outputs an image corresponding to the understanding level of the user. Accordingly, the tone and image information 53 includes tones and images corresponding to the understanding levels of users.

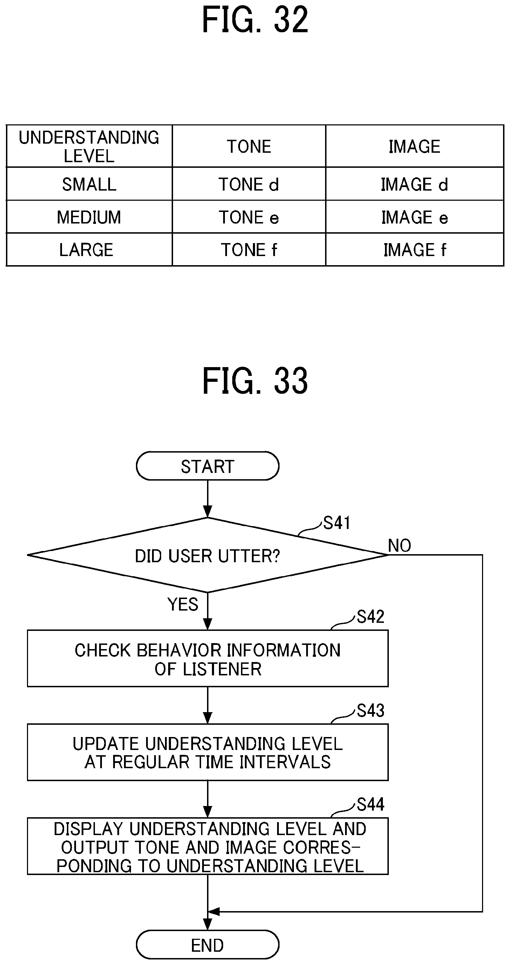

FIG. 32 is a diagram illustrating a data structure of the tone and image information 53 according to the present embodiment. The tone and image information 53 in FIG. 32 includes items of understanding level, tone, and image.

The item of understanding level represents the understanding level determined by the user state determination unit 41.

The item of tone represents tone associated with the understanding level. For example, when the understanding level is low, a tone that increases the concentration is used, and when the understanding level is high, a tone that promotes more creative output is used.

The item of image represents an image or video associated with the understanding level. For example, when the understanding level is low, an image that increases the concentration is used, and when the understanding level is high, an image that promotes more creative output is used.

The second environmental control unit 37 may display the understanding level 250 on the image display device 12 closest to the two users A and B. Alternatively, the second environmental control unit 37 may display the understanding level 250 on the image display device 12 facing the user who has uttered words.

FIG. 33 is a flowchart of a process of displaying the understanding levels of users having a face-to-face conversation, performed by the second environmental control unit 37 according to the present embodiment. The description with reference to FIG. 33 is on the assumption that the user group determination unit 32 has already determined that the users face each other.

The user state determination unit 41 determines whether the user has uttered (S41). Which user has uttered words is identified by the user ID transmitted by the activity sensor 9. Further, another user facing the user who has uttered words is identified as the listener.

When the user utters words, the user state determination unit 41 checks the behavior information of the user being the listener with respect to the utterance (S42). It is preferable that the user state determination unit 41 focuses on the behavior information of the listener from the start to the end of the speech data and within a certain time from the end. The user state determination unit 41 records the behavior information together with time.

The user state determination unit 41 deducts the number of times of head tilt from the number of times of nodding in a certain past time and updates the understanding level based on the calculated value (S43). The user state determination unit 41 may weight the number of times of nodding or head tilt immediately after the utterance. For example, the number of times of each nodding or head tilt immediately after the utterance is considered to be larger than 1. This is because there is a high probability that the nodding or head tilt immediately after the utterance reflects the understanding level.

The second environmental control unit 37 displays the understanding level of the user on the image display device 12, the first environmental control unit 36 outputs a tone corresponding to the understanding level, and the second environmental control unit 37 outputs an image corresponding to the understanding level (S44). Further, the second environmental control unit 37 may display the history of the understanding level of each user.

Displaying the understanding level of the user in this manner helps the speaker speak so as to be easily understood by the listener. Further, the information processing system 10 outputs a tone or an image to increase the understanding level.

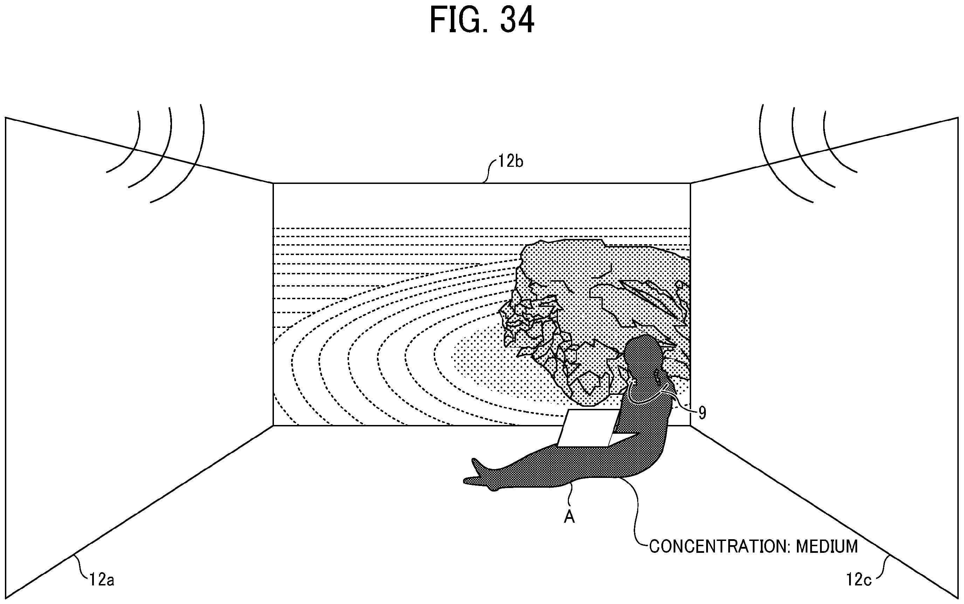

With reference to FIG. 34, a description is given below of a method of determining a concentration level as another user state, according to the present embodiment. FIG. 34 is a diagram illustrating a method of determining a concentration level as another user state, according to the present embodiment. In FIG. 34, the user A is working alone. The user state determination unit 41 detects, for each user, changes in the position and the direction and determines the concentration level of the user in a meeting or a personal work. The first environmental control unit 36 and the second environmental control unit 37 perform environmental control according to the concentration level of the user.

In a case where the position and the direction hardly change and the user A is in a predetermined posture, the user state determination unit 41 determines that the user A should concentrate and determines the concentration level. Examples of the predetermined posture include sitting positions such as full-lotus sitting, cross-legged sitting, and seiza. Seiza is kneeling with the legs folded underneath the thighs and the buttocks resting on the heels, with ankles turned outward. For example, when the user state determination unit 41 determines that the user does not move but his/her head is inclined down based on the behavior information, the user state determination unit 41 determines that the user is drowsy and the concentration level is low.

The user state determination unit 41 determines a user state other than the understanding level and the concentration level. For example, the user state determination unit 41 determines the strength of the impact received by the user based on a face-up posture. The user state determination unit 41 determines a state of anxiety or depression based on a face-down posture. The first environmental control unit 36 and the second environmental control unit 37 perform appropriate environmental control according to the state of the user.

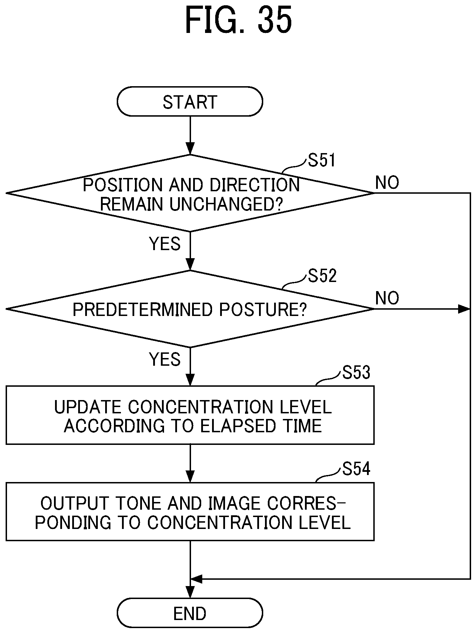

FIG. 35 is a flowchart of a process of environmental control in accordance with the concentration level, according to the present embodiment. The user may stay alone or face another user.

The user state determination unit 41 determines whether the position and the direction of the user remain unchanged (S51). In a case where the user is standing, the user may move slightly even if the user concentrates on work. Accordingly, the user state determination unit 41 may ignore changes within a certain degree of the position or the direction.

When the determination in step S51 is Yes, the user state determination unit 41 determines whether the user is in a predetermined posture on the basis of the behavior information of the user (S52). For example, when the behavior information indicates that the user is sleeping or doing exercises, the user state determination unit 41 determines that the user does not concentrate on work. When the determination of S51 is No, the process of FIG. 35 ends.

In a case where the determination in step S52 is Yes, the user state determination unit 41 updates the concentration level in accordance with the elapsed time from when the user took the predetermined posture without changing the position and the direction (S53). When the determination of S52 is No, the process of FIG. 35 ends.

The first environmental control unit 36 outputs a tone corresponding to the concentration level, and the second environmental control unit 37 outputs an image corresponding to the concentration level (S54). Further, the second environmental control unit 37 may display the history of the concentration level of each user.

The tone or image corresponding to the concentration level is a tone or image for increasing (and maintaining) the concentration level. Alternatively, the tone or image corresponding to the concentration level is a tone or image for reporting a decrease in the concentration level when the concentration level decreases. The second environmental control unit 37 may display the image on the image display device 12 closest to the user A or the image display device 12 positioned in the face direction of the user A. Alternatively, the second environmental control unit 37 may display the image corresponding to the concentration level on all the image display devices 12.

In this way, the information processing system 10 outputs a tone and an image to increase and maintain the concentration level.

### Display of Cursor

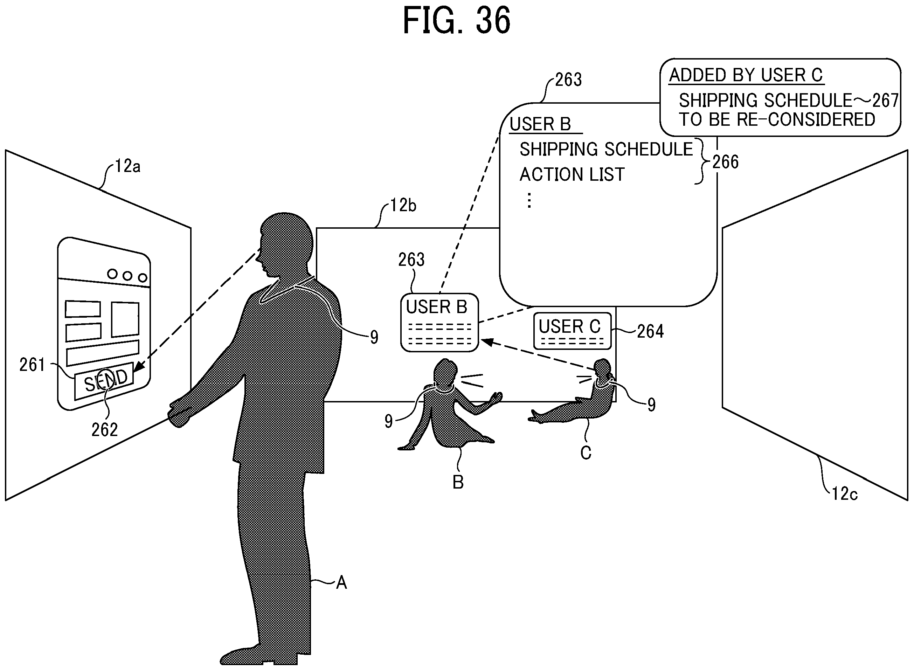

A description is given below of the display of a cursor using the behavior information, with reference to FIG. 36. FIG. 36 is a diagram illustrating a method of displaying a cursor and usage of the cursor according to the present embodiment. In FIG. 36, the user A is near the image display device 12a and faces the image display device 12a. Accordingly, the work area of the user A is displayed on the image display device 12a. The cursor position determination unit 42 determines to display a cursor on the image display device 12a positioned in the direction of the user's face. The direction of the user's face is the direction to which the user directs his/her face. The direction of the user's face (may be referred to as "face direction" in the following description) is included in the behavior information.

The second environmental control unit 37 preferably places a cursor 262 on, for example, a button 261 included in the image in the direction of the user's face. In other words, even if the direction of the user's face is slightly deviated from the position of the button 261, the second environmental control unit 37 can enhance the operability by forcibly positioning the cursor 262 on the button 261. When the user utters words such as "press" or "on," under such conditions, the information processing system 10 detects the pressing of the button 261 from the speech data. Accordingly, the information processing system 10 allows the user to manipulate the image based on the direction of the user's face and voice.

In FIG. 36, the users B and C face each other and have a conversation. The users B and C are grouped.

The users B and C are guided to the image display device 12b. Accordingly, the second environmental control unit 37 displays work areas 263 and 264 of the users B and C on the image display device 12b. In such a case, the user C can add speech data to the work area of the user C based on his/her face direction. Specifically, the information processing system 10 adds speech data obtained from speech recognition on signals acquired via the communication unit 31 from, for example, the microphone 20 to the work area determined from the face direction included in the behavior information. The cursor position determination unit 42 detects the work area 263 in the face direction of the user B and adds speech data 267 uttered by the user C to speech data 266 in the work area 263. The speech data 267 added to the work area 263 may be highlighted with, for example, a different color. In FIG. 36, the work area 263 of the user B is illustrated in an enlarged manner for convenience of explanation.

Needless to say, the user B can also add speech data to the work area 264 of the user C. Since the information processing system 10 allows the addition of the speech data only among the grouped users, an unrelated user is prevented from adding speech data.

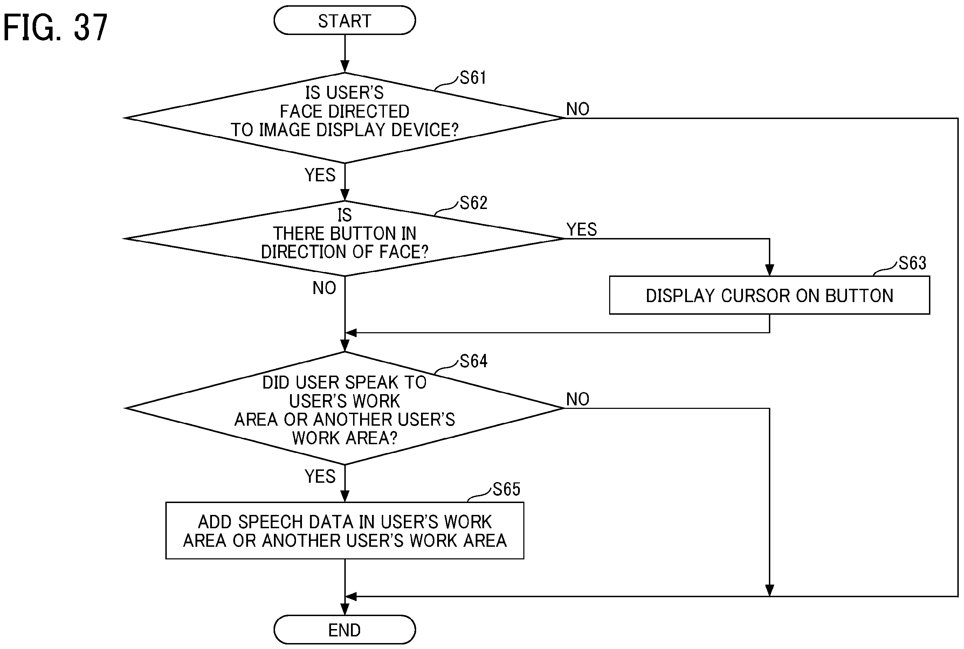

FIG. 37 is a flowchart of a method of displaying a cursor and usage of the cursor according to the present embodiment.

The cursor position determination unit 42 determines whether or not the user faces the image display device 12 (S61).

When the determination in step S61 is Yes, the cursor position determination unit 42 determines whether or not there is a button in the direction of the user's face (S62). When the determination of S61 is No, the process of FIG. 37 ends.

If the determination in step S62 is Yes, the cursor position determination unit 42 determines to align the cursor position with the button (S63). The second environmental control unit 37 displays a cursor superimposed on the button.

Subsequently, the cursor position determination unit 42 determines whether or not the user has spoken while facing the work area (S64). This work area may be the user's own work area or another user's work area.

When the determination in step S64 is Yes, the second environmental control unit 37 additionally displays the speech data in the user's own work area or another user's work area based on the direction in which the user has uttered words (S65).

In this way, the information processing system 10 determines the face direction of the user relative to the image display device 12 and controls the environment such as the image.

### Office Optimization

The tendencies of the levels of concentration and relaxation of users and conversation volume may depend on the location in the office. In other words, it is possible that the usage rate of a certain place is high and the levels of concentration and relaxation of users and conversation volume there are high, while the usage rate of another place is low and the levels of concentration and relaxation of users and conversation volume there are low. It can be said that the convenience of the former place is high but the convenience of the latter place is low. However, as an office, it is preferable that convenience is high at any place. A description is given of a method of summarizing the degrees of convenience of the places determined based on these indices and displaying the degree of convenience in association with the place. This allows the manager of the organization or the administrator of the office to optimize the layout of the office to increase the convenience.

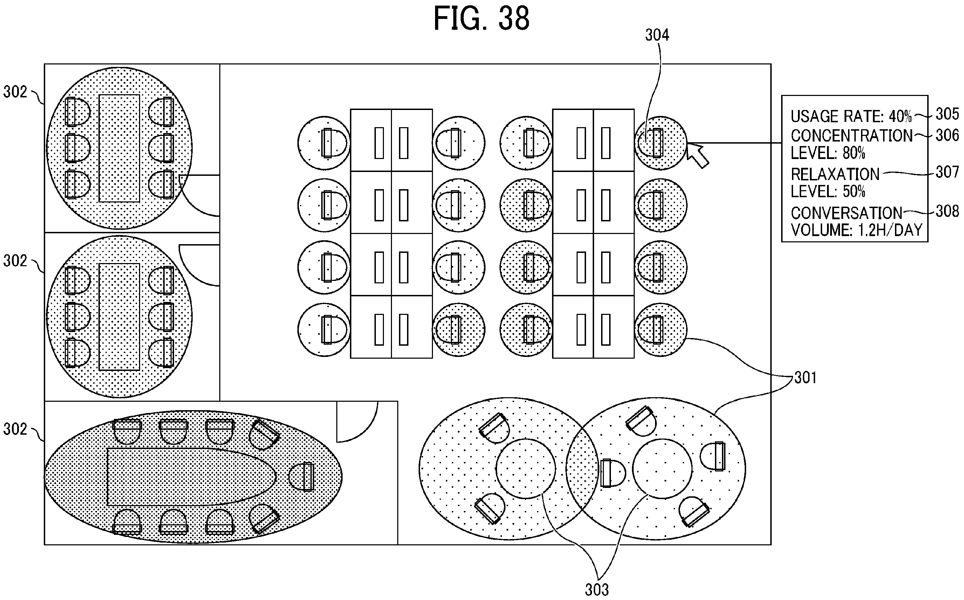

FIG. 38 is a diagram illustrating correspondence between the layout of an office and the convenience according to the present embodiment. The convenience is obtained by quantifying the usage rate, the levels of concentration and relaxation of users, and the conversation volume of a location of the office. One or more of these indices are not necessarily used. These indices may be replaced with equivalent indices. For example, the level of relaxation can be substituted by the level of psychological safety.

In FIG. 38, a place with higher convenience is indicated with a darker shading pattern. The darkness of the shading pattern indicates that the convenience of a meeting room 302 is high, but the convenience of a table 303 in the open space is low. In such a case, the manager of the organization can, for example, move or remove the table 303 having low convenience.

The convenience for each place in FIG. 38 can be displayed by the information processing terminal 22. In this case, when the manager, the administrator, or the user moves the cursor to or clicks a particular place with a mouse, the information processing terminal 22 displays a usage rate 305 of the place, a concentration level 306 of users, a psychological safety level (relaxation degree) 307, and a conversation volume 308. For example, in FIG. 38, the information processing terminal 22 displays a usage rate of 40%, a concentration level of 80%, a relaxation level of 50%, and a conversation volume of 1.2h/day. The user can select a meeting room or a desk to be used by viewing such information.

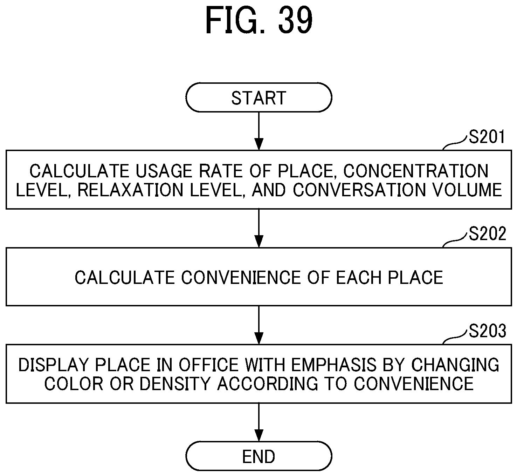

FIG. 39 is a flowchart of a process of presenting the conveniences of places in an office, according to the present embodiment. The process of FIG. 39 can be performed in real time in response to a request made by, for example, the manager, the administrator, or a user via the information processing terminal 22.

The convenience presentation unit 45 calculates the usage rate of each place; and the concentration level, the relaxation level, and the conversation volume in each place (S201). These indices are calculated based on the activity information of, for example, a most recent period (for example, one month). Examples of the place include meeting rooms, seats, and tables. The convenience presentation unit 45 acquires the position information of users in these places from the activity information, and calculates the usage rate, the concentration level, the relaxation level, and the conversation volume.

The usage rate is obtained by averaging, for example, the percentage of time of use of the place per day in a certain period. The concentration level is obtained by calculating the percentage of the time during which it is determined that the user is concentrated to the time during which the user is in the place and averaging the calculated percentages in a certain period. The relaxation level is obtained by calculating the percentage of the time during which it is determined that the user is relaxed to the time during which the user is in the place and averaging the calculated percentages in a certain period. Whether or not the user is relaxed is determined from the heartbeat detected by the activity sensor 9. The time-series data of heartbeat interval variation is represented by R-R interval (RRI) signals, and attention is paid to a high frequency-component (HF) and a low-frequency component (LF) of the RRI signals. It is known in the art that the HF is an activity index of the parasympathetic nerve and thus increases when the subject is relaxed, and the LF is an activity index of the sympathetic nerve and thus increases when the strain of the subject increases. Accordingly, a method of calculating the relaxation level from the expression HF/(HF + LF) is known in the art. The conversation volume is obtained by calculating, for example, the total of time during which it is determined that users have conversations in one day at the place and averaging the total in a certain period.

The convenience presentation unit 45 calculates the convenience of each location (S202). The convenience presentation unit 45 calculates the degree of convenience by, for example, weighting the usage rate, the concentration level, the relaxation level, and the conversation volume.

The convenience presentation unit 45 transmits, to the information processing terminal 22, screen information for a screen in which places of the office are highlighted with differences in color or shading pattern density according to the degree of convenience (S203). The information processing terminal 22 displays a screen on which the convenience is presented for each office place as illustrated in FIG. 38. The convenience presentation unit 45 may display the convenience of the place when the user approaches the image display device 12 near that place.

As described above, the convenience presentation unit 45 summarizes the degree of convenience based on the activity information, to optimize the office design. This helps the user to select and use a highly convenient place.

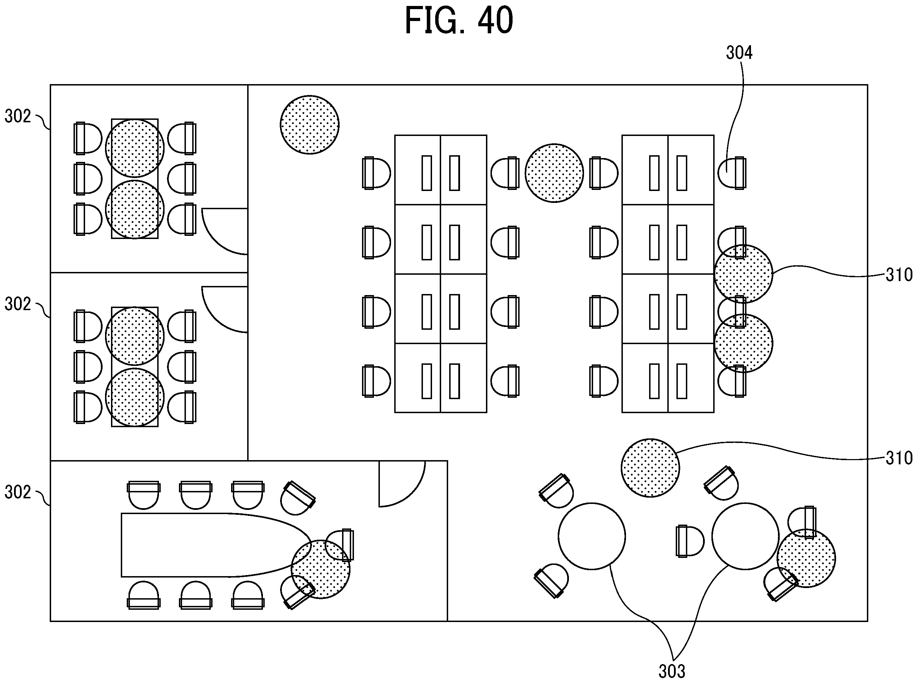

Further, using the activity information, the convenience presentation unit 45 can determine a place where meaningful communications are to be performed in the office. The place where meaningful communications are to be performed is a place where communications are easily performed, which is described below with references to FIG. 40.

FIG. 40 is a diagram illustrating the determination of a place where meaningful communications are likely to be performed in an office, according to the present embodiment. In FIG. 40, places location where meaningful communications are likely to be performed are indicated with marks 310. The information processing terminal 22 can display the ease of communication for each place in FIG. 40. In this case, when the manager, the administrator, or the user moves the cursor to or clicks a particular place with a mouse, the information processing terminal 22 displays a score of the ease of communication.

Whether or not meaningful communications are performed may be determined from, for example, the number of nods, the voice volume of speech data, and heartbeat. One or more of these indices are not necessarily used. These indices may be replaced with equivalent indices. For example, the number of nods may be substituted with positive speech, and the heartbeat may be substituted with pulse.

The number of nods is obtained by averaging, for example, the frequency (the number of times per unit time) of nods in communication in the place in a certain period. Since communication is performed among multiple users, the number of times of nodding by all users in communication is counted. The voice volume of the speech data is obtained by, for example, averaging the voice volume of speech of the user in a certain period in the place. The heartbeat is obtained by, for example, averaging the heartbeat of the user in a certain period at the place. The larger the amount of nodding, the voice volume of speech data, and the heartbeat, it can be determined that meaningful communications are performed. The communicative level presentation unit 46 weights the number of nods, the sound volume of speech data, and the heartbeat to quantify the ease of communication.

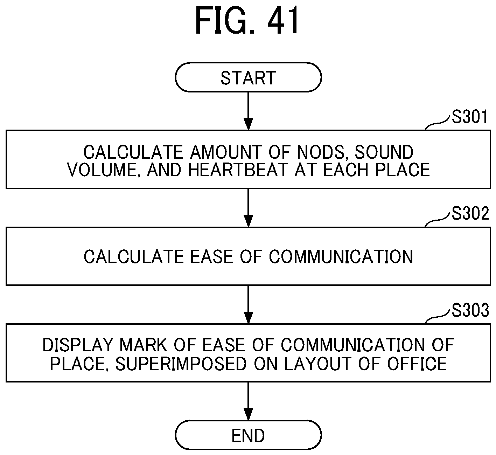

FIG. 41 is a flowchart of a process of presenting a place where meaningful communications are performed in an office layout, according to the present embodiment. The process of FIG. 41 can be performed in real time in response to a request made by, for example, the manager, the administrator, or a user via the information processing terminal 22.

The communicative level presentation unit 46 calculates the number of nods, the sound volume, and heartbeat at each place (S301). These indices are calculated based on the activity information of, for example, a most recent period (for example, one month). The place is specified based on the position information of two or more users who have communicated with each other. In other words, the communicative level presentation unit 46 regards the grouped users as being communicating, and specifies, as the place, for example, a circular or rectangular range surrounding the position information of these users or a circle having a predetermined radius from the center of gravity of the position information of these users. The place may be specified by the unit of, for example, meeting room, seat, and table.

The communicative level presentation unit 46 calculates the ease of communication at each place (S302). For example, the convenience presentation unit 45 calculates the ease of communication by weighting the number of nods, the sound volume, and the heartbeat.

The communicative level presentation unit 46 transmits, to the information processing terminal 22, a screen that displays a mark having a color or density corresponding to the ease of communication, superimposed on the layout of the office (S303). The information processing terminal 22 displays a screen on which the ease of communication is presented for each office place as illustrated in FIG. 40. The communicative level presentation unit 46 may display the ease of communication of the place when the user approaches the image display device 12 near that place.

As described above, since the communicative level presentation unit 46 presents a place where meaningful communications are performed, the user can select the place where meaningful communications are performed to have a communication.

The environmental control system 100 of the present embodiment may control the environment of the meeting room by using the position information, the direction information, and the behavior information of the user.

### Embodiment 3

In the present embodiment, a description is given of environmental control in a virtual meeting on the assumption that a user carrying the activity sensor 9 participates in the virtual meeting.

Some technologies are known in the art to display a screen of an electronic whiteboard or a PC screen in a virtual space, and enable users to hold, for example, a meeting while viewing a screen displaying a virtual space with virtual reality (VR) goggles. Some technologies are known in the art to hold, for example, online meetings in which a PC monitor displays the faces of the participants or a screen of an electronic whiteboard. Compared with such online meetings, in the virtual space (within the field of view of the VR goggles), the participants and the screen of the electronic whiteboard or PC are displayed as if the participants and the screen are in front of the eyes. Accordingly, the participants can feel the realism of the meeting.

Regarding the environmental control according to Embodiments 1 or 2, since an image can be displayed on, for example, any wall, ceiling, or floor in the virtual space, the degree of freedom of the environmental control is expected to increase.

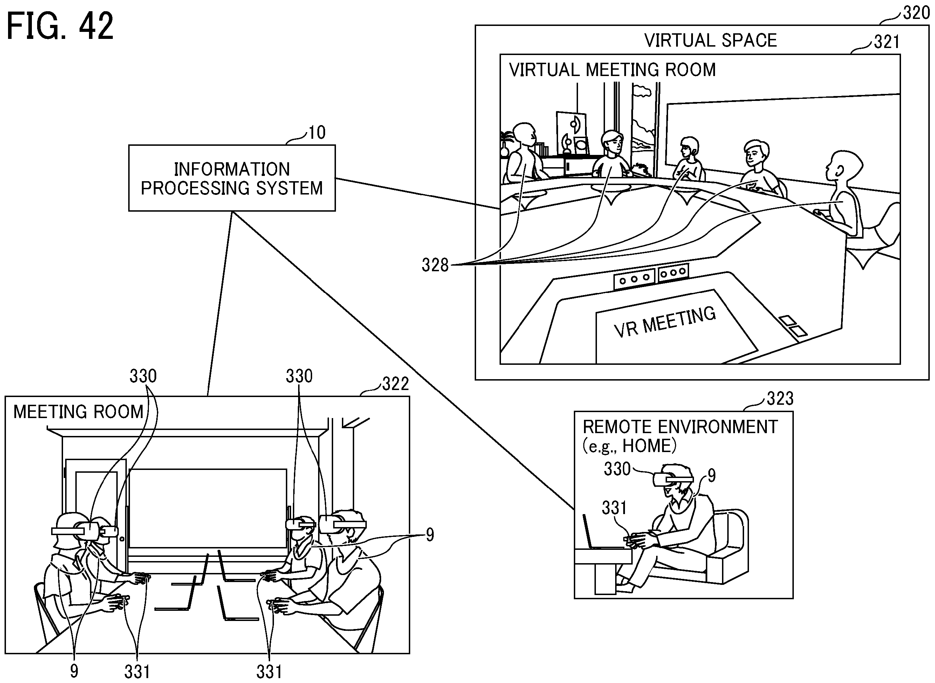

FIG. 42 is a diagram illustrating a system configuration of the information processing system 10 that generates a virtual space 320 according to the present embodiment. The information processing system 10 constructs the virtual space 320 on a memory and executes environmental control in the virtual space 320. The virtual space 320 is, for example, a 360-degree three-dimensional space that imitates a space or a meeting room where people gather in a virtual space. For example, VR goggles or a VR headset (referred to as a display terminal 330 in the following description) worn by a user displays a field of view of the user in a virtual space. Further, an avatar 328 of the user is present in the virtual space 320. The field of view of the user is an image obtained by projecting a three-dimensional space onto a virtual camera on the assumption that the optical axis of the virtual camera is located in the line-of-sight direction from the position of the avatar 328. The user who participates in the meeting can change the position of the avatar 328 and the direction (including the line-of-sight direction) of the avatar 328 by operating a controller 331 for VR operation (may be referred to simply as "controller 331" in the following description). Further, the user can change the direction of the avatar 328 by operating the controller 331 or by changing his/her direction. In other words, the direction of the avatar 328 can be changed based on the direction information detected by the activity sensor 9. Accordingly, the user can view any viewpoint of the virtual space 320.

As described in Embodiments 1 and 2, the information processing system 10 communicates with the activity sensor 9 of each user. In FIG. 42, for example, four users are present in a meeting room 322, and one user is in a remote environment 323 such as a home. Each user wears the activity sensor 9 on his/her neck. Accordingly, the activity information of each user is transmitted to the information processing system 10.

In the present embodiment, the position information of the users in the meeting room 322 transmitted to the information processing system 10 may indicate the respective locations of the users in the meeting room 322.

However, when the virtual space 320 is the virtual meeting room 321 as illustrated in FIG. 42, the position information detected by the activity sensor 9 may not be used for the position of the avatar 328. This is because the user does not move in the meeting room 322 during the meeting, and the layout of the fixtures in the meeting room 322 does not match the layout of the fixtures in the virtual meeting room 321. However, if there are no such restrictions, the position information of the users in the meeting room 322 may be reflected in the locations of the avatars 328 in the virtual meeting room 321.

The user operates the controller 331 carried by the user for VR operation to move the avatar in the virtual space 320. In the initial state, the direction of the avatar in the virtual space 320 matches the direction of the seat in the virtual space 320. The direction of the avatar is directed to the direction instructed by the operation of the controller 331 or the direction detected by the activity sensor 9. Thus, the face direction in the virtual space 320 is specified.

In the virtual meeting room 321, the user participating from the meeting room 322 or the remote environment 323 can view other participants and the screens of the devices installed in the meeting room as if the user is in a real meeting room.

In the case of the system configuration illustrated in FIG. 42, examples of the display terminal 330 include VR goggles, a VR headset, and augmented reality (AR) goggles, mixed reality (MR) goggles, and a head mounted display (HMD). These display terminals 330 change the field of view in the virtual space 320 in conjunction with the change in the user's line-of-sight direction, and display the image in this field of view. Alternatively, the display terminal 330 may be, for example, a PC or a smartphone. In this case, the image displayed in conjunction with the line-of-sight direction of the user is not changed, but the user can operate, for example, the PC or the smartphone, to change the field of view in the virtual space 320 and display the image in the field of view.

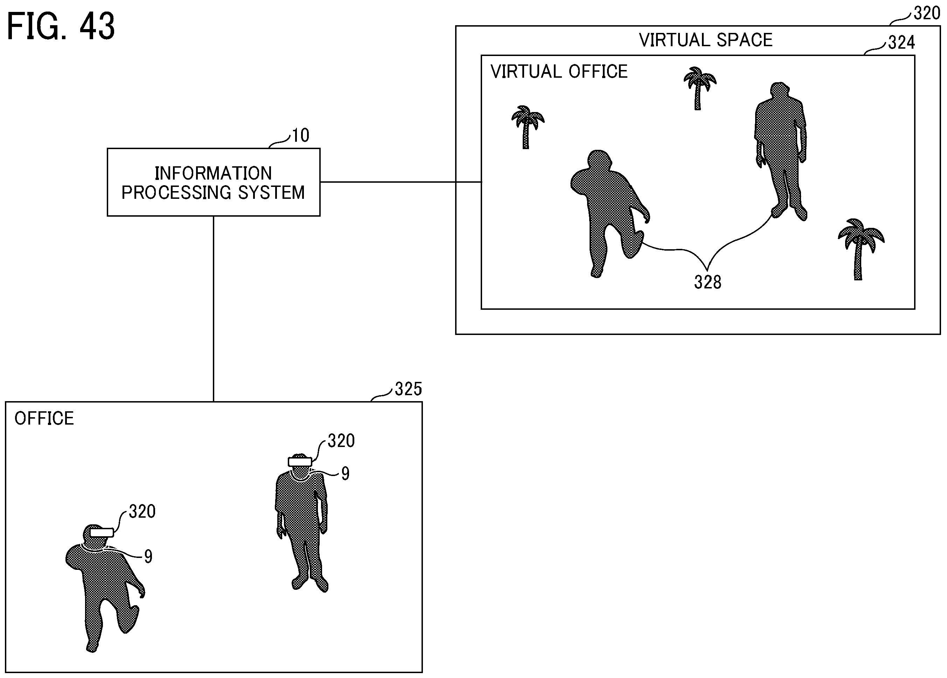

FIG. 43 is a diagram illustrating a system configuration of the information processing system 10 that generates a virtual office 324 as the virtual space 320, according to the present embodiment. In the system configuration of FIG. 43, the position information of the activity sensor 9 may be reflected in the position of the avatar in the virtual space 320. In other words, the virtual office 324 is not limited to a virtual meeting room but may be any virtual space. The virtual office 324 may be, for example, a space that is equivalent in shape and area to a real office 325 and is provided with, for example, a virtual display. Alternatively, the virtual office 324 may be equivalent in shape to the real office 325 but narrower or wider.

Generally, it is difficult for a user wearing VR goggles to perform an activity while freely walking in a real space with many obstacles since the field of view is obstructed by the VR goggles. In the related art, if the obstacle is a fixture, which does not move, the three-dimensional data of the obstacle relative to the space is obtained in advance and an obstacle having the same size is reproduced at the same position in a virtual space based on the three-dimensional data, to prevent collision in the real space. However, this method is not applicable for an object such as a person that moves freely. By contrast, in the present embodiment, since the position information of a person is acquired and processed by the information processing system 10, the information processing system 10 reproduces the person in a virtual space in the same or substantially same positional relation as in a real space. Accordingly, the collision between persons in the virtual space can be prevented. As a result, even when the fields of view are fully obstructed by the VR goggles, the persons can freely move around.

When the virtual space 320 is the virtual office 324 as illustrated in FIG. 43, the position information and the direction information detected by the activity sensor 9 are used for the position and the direction in the virtual office 324. The information processing system 10 reproduces the real office 325 in the real space in the virtual space 320, and uses the position information and the direction information detected by the activity sensor 9 for the position and the direction of the avatar 328. As the user moves in the office 325, the avatar 328 also moves in the virtual office 324. The same applies to the direction. Accordingly, the controller 331 is not used to control the position and direction of the avatar 328, but may be used or may be separately used for, for example, a user interface operation.

In FIG. 43, the information processing system 10 may allow a user to participate in the virtual office 324 from a meeting room or a remote environment.

### Hardware Configuration

### VR goggles

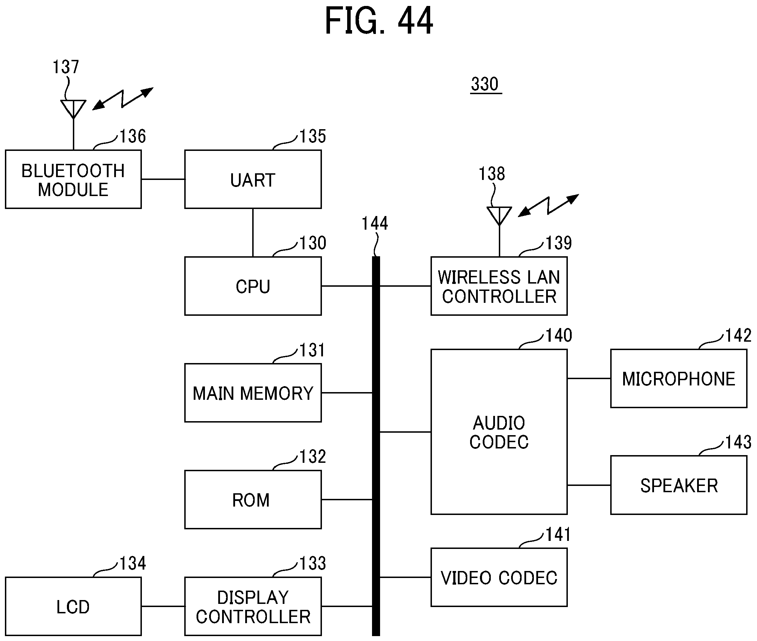

FIG. 44 is a block diagram illustrating a hardware configuration of the display terminal 330 according to the present embodiment. The display terminal 330 includes a CPU 130, a main memory 131, a ROM 132, a display controller 133, a wireless LAN controller 139, an audio codec 140, and a video codec 141, which are connected with each other via a bus 144.

The CPU 130 executes an operating system (OS) and a control program read from the ROM 132 to the main memory 131, to perform various types of processing. The main memory 131 includes a dynamic RAM (DRAM), and is used as, for example, the work area of the CPU 130.

In the ROM 132, the OS, a system program at power on, and a program for controlling the display terminal 330 are written in advance.

To the CPU 130, a universal asynchronous receiver-transmitter (UART) 135 is connected. The UART 135 is an interface for serial data transmission and reception between the CPU 130 and a BLUETOOTH module 136, and includes, for example, a first-in first-out (FIFO) memory and a shift register.

The BLUETOOTH module 136 includes a radio frequency (RF) unit and a baseband unit and is connected to an antenna 137. The BLUETOOTH module 136 performs wireless communication conforming to BLUETOOTH protocols.

The display controller 133 performs digital-to-analog (D/A) conversion on, for example, text, graphics, and image data, and performs control for displaying these data on a liquid crystal display (LCD) 134.

The wireless LAN controller 139 executes a communication protocol conforming to the Institute of Electrical and Electronics Engineers (IEEE) 802.11ax, and controls communication with other devices by transmitting and receiving radio waves via the antenna 138.

A sound signal received from the microphone 142 is converted into sound signal by an analog-to-digital (A/D) conversion circuit, and the sound data is encoded by an advanced audio coding (AAC) method by the audio codec 140. AAC encoded data received from an external device is decoded by the audio codec 140, converted into an analog signal by the D/A conversion circuit, and output from a speaker 143. A video codec 141 decodes compressed video data received from an external device. The compressed video data is in the format in conformity with, for example, the International Telecommunication Union (ITU)-T Recommendation H.264. Data is exchanged between the above-described components via the bus 144.

### Controller for Operating VR

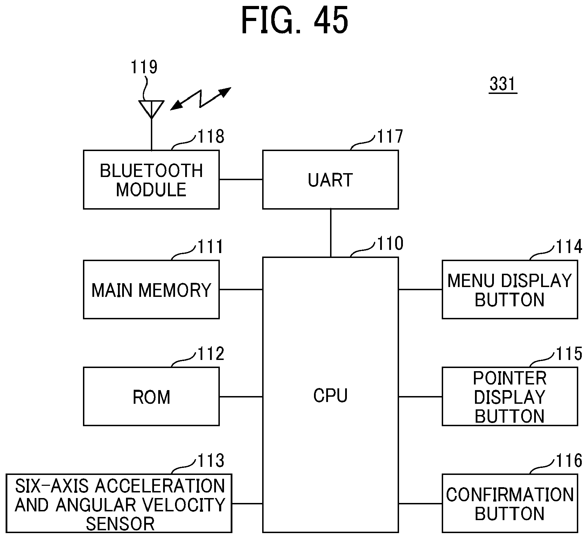

FIG. 45 is a block diagram illustrating a hardware configuration of the controller 331 according to the present embodiment. The controller 331 includes a UART 117, a main memory 111, a ROM 112, a six-axis acceleration and angular velocity sensor 113, a menu display button 114, a pointer display button 115, and a confirmation button 116, which are connected to a CPU 110.

The CPU 110 executes a control program read from the ROM 112 to the main memory 111, to perform control processing. The main memory 111 includes a DRAM and is used as, for example, a work area of the CPU 110.

In the ROM 112, a system program at the time of power-on and a program for transmitting information on pressing of the menu display button 114, the pointer display button 115, and the confirmation button 116 by BLUETOOTH are written in advance.

The six-axis acceleration and angular velocity sensor 113 outputs measurement data of acceleration and angular velocity. The UART 117 is an interface for serial data transmission and reception between the CPU 110 and a BLUETOOTH module 118, and includes, for example, a FIFO memory and a shift register. The BLUETOOTH module 118 includes an RF unit and a baseband unit and is connected to an antenna 119. The BLUETOOTH module 118 performs wireless communication conforming to BLUETOOTH protocols.

### Functions

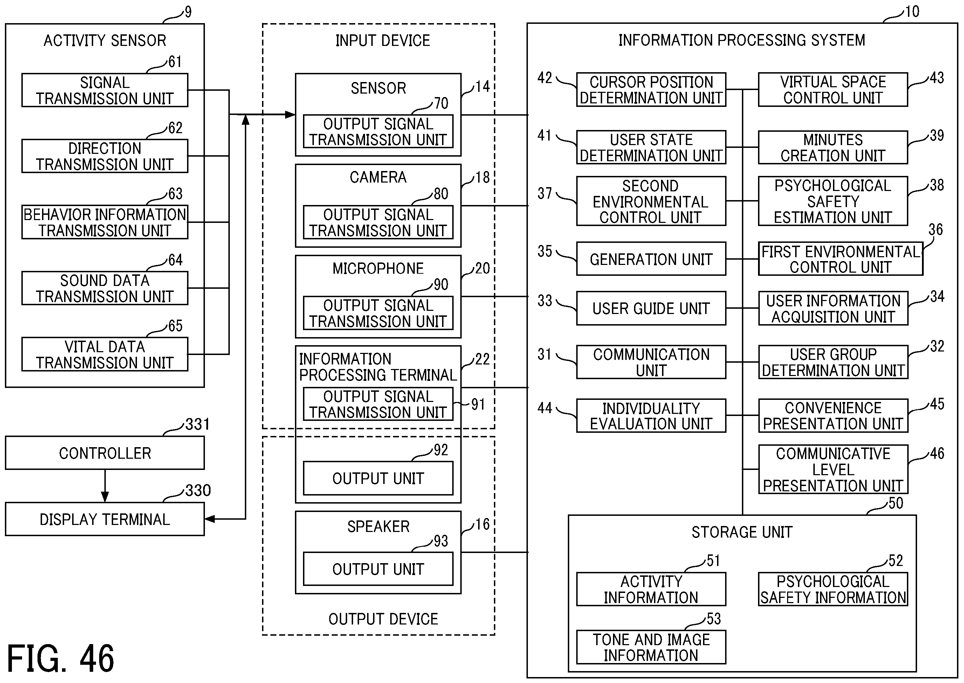

FIG. 46 is a block diagram illustrating a functional configuration of the information processing system 10 according to the present embodiment. Like reference signs are given to elements in FIG. 46 similar to those illustrated in FIG. 30 as those elements operate similarly and provide the same or similar effects, and redundant descriptions may be omitted in the following description.

The information processing system 10 of FIG. 46 includes a virtual space control unit 43. The virtual space control unit 43 stores in a memory, for each virtual space 320, the display terminals 330 and the activity sensors 9 to be connected in association with the identification information of the virtual space 320. Further, the virtual space control unit 43 stores in a memory the display terminal 330 and the activity sensor 9 worn by the users in association with the user ID. Thus, the activity information such as the position information, the direction, and the speech data of the user is associated with the display terminal 330.

The first environmental control unit 36 of the present embodiment outputs a tone (ambient sound) corresponding to the sound data generated by the generation unit 35 in the virtual space 320. There are two manners of output. One is outputting the tone from all the display terminals 330 so that all avatars in the virtual space 320 hear the tone. The other is outputting the toner from only the display terminal 330 of the specific avatar so that only the specific avatar hears the tone.

The second environmental control unit 37 outputs an image corresponding to the image data generated by generation unit 35 to virtual space 320. There are two manners of output. One is outputting the image to all the display terminals 330 so that all avatars in the virtual space 320 can view the image. The other is outputting the image to only the display terminal 330 of the specific avatar so that only the specific avatar can view the image.

### Display Terminal

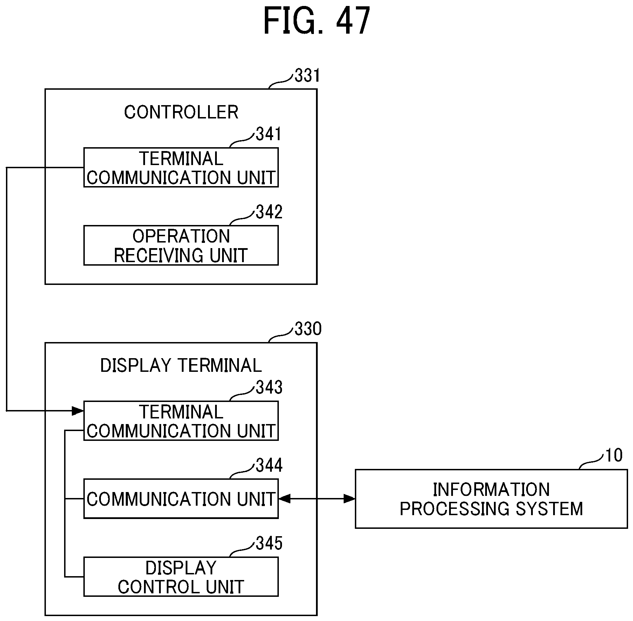

With reference to FIG. 47, a description is given of a functional configuration of the display terminal 330 according to the present embodiment. FIG. 47 is a block diagram illustrating functional configurations of the display terminal 330 and the controller 331 according to the present embodiment. The display terminal 330 includes a terminal communication unit 343, a communication unit 344, and a display control unit 345.

The terminal communication unit 343 communicates with the controller 331 by wireless communication such as BLUETOOTH and receives operation information from the controller 331. The communication unit 344 transmits, for example, the operation information received from the controller 331 to the information processing system 10 via an input device or directly. Further, the communication unit 344 receives, from the information processing system 10, an image of the entire virtual space 320 or a part thereof corresponding to a partial field of view range. The display control unit 345 displays an image of the virtual space 320 in the field of view range corresponding to the position and the direction of the avatar.

### Controller

A description is given of a functional configuration of the controller 331 according to the present embodiment. The controller 331 includes a terminal communication unit 341 and an operation receiving unit 342. The operation receiving unit 342 receives a user's operation on the controller 331 (such as pressing of a button for instructing a position or a direction, or pressing of a button for selecting a menu). The terminal communication unit 341 communicates with the controller 331 by wireless communication such as BLUETOOTH and transmits the operation information to the controller 331.

### Virtual Space Control Unit

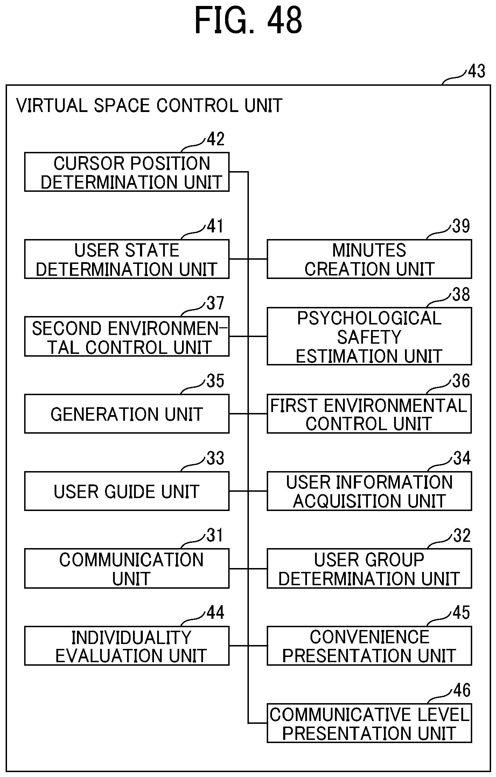

FIG. 48 is a block diagram illustrating in detail a functional configuration of the virtual space control unit 43 according to the present embodiment. The virtual space control unit 43 executes the environmental control of Embodiment 1 or 2 in the virtual space 320. Accordingly, the information processing system 10 implements the functions in Embodiment 1 or 2 in the virtual space 320. The following description with reference to FIG. 48 is focused on the differences from FIG. 30.

The user information acquisition unit 34 converts the activity information of the user received by the communication unit 31 from the sensor 14 to the activity information in the virtual space 320, and stores the activity information in time series for each user. The term "conversion" used here relates to position information. In the system configuration of FIG. 42, "conversion" refers to conversion into position information of a seat in the virtual meeting room 321 instead of position information of the meeting room 322, and conversion into position information designated by the controller 331. In the system configuration of FIG. 43, when the office 325 and the virtual office 324 are equivalent in size and shape, no conversion is required. Otherwise, the position information of the office 325 may be converted to the position information of the virtual office 324, with, for example, mapping information that converts the position information of the office 325 to the position information of the virtual office 324.

The activity information has a data structure similar to that in FIG. 8, but the activity information relating to virtual space control represents activities in the virtual space 320. As for the position information, in the system configuration of FIG. 42, the position information detected by the activity sensor 9 is not used. Instead, for example, the position information of the seat assigned to the avatar in the virtual space 320 by the virtual space control unit 43 is used, or the position information received by the controller 331 is used. In the system configuration of FIG. 43, for example, the position in the real space detected by the activity sensor 9 is reflected in the position of the virtual space 320. As for the direction, the direction detected by the activity sensor 9 is reflected in both cases of FIGS. 42 and 43. Further, the direction may be operated by the controller 331.

Further, in the present embodiment, the following activity information can also be acquired.

Avatar information: The avatar information is information of the avatar used by the user in the virtual space 320. The avatar information includes information on the position and the direction of the avatar in the virtual space 320. The avatar information further includes information on, for example, appearance, clothes, and accessories of the avatar.

Action history: The action history is a history of actions performed by the avatar in a metaverse. For example, the action history includes places the avatar has visited and the activities (e.g., a meeting or a conversation) the avatar has performed.

Communication data: The communication data is data of, for example, text chats, voice chats, and gestures with which the avatar communicates with another avatar.

Interests and hobbies information: Interests and hobbies information related to hobbies and preferences of the avatar and indicates, for example, a content or activity in which the avatar is interested and places the avatar frequently visits.

Social graph: A social graph is information that visually represents the relation of the avatar with another avatar (friendship or belonging to a group) by, for example, connection lines or color coding.

Sensor data: Sensor data is information related to a physical motion or state of an avatar obtained using a sensor (such as a position sensor or an acceleration sensor) mounted on the display terminal 330.

Advertisement or personalization: Advertisement or personalization is information for individually customizing or recommending an advertisement or content based on the action history or the interests of the avatar.

### Environmental Control in Virtual Space

Descriptions are given below of environmental control in the virtual space 320 based on the flowcharts of FIGS. 15, 22, 25, 27, and 29 of Embodiment 1; and flowcharts of FIGS. 33, 35, 37, 39, and 41 of Embodiment 2.

### Output of Tone or Image corresponding to Psychological Safety Level

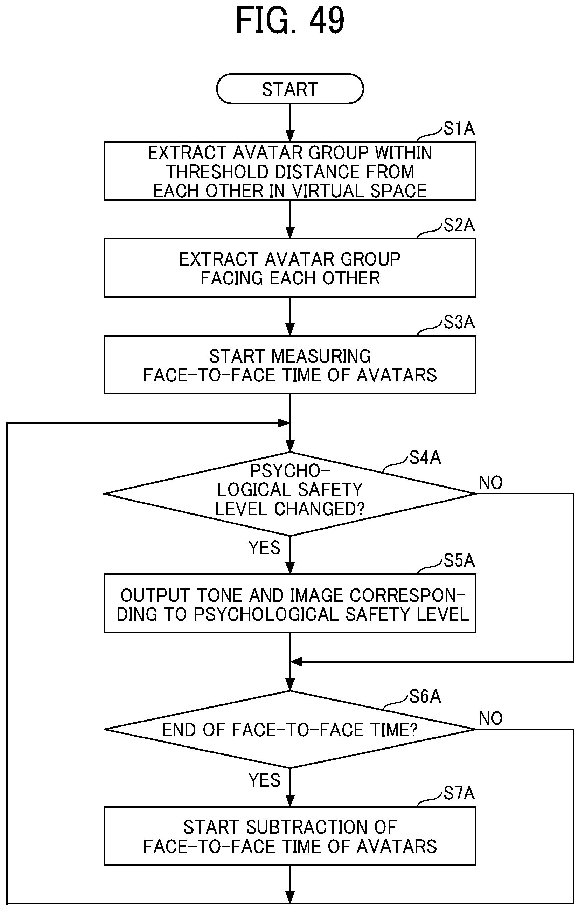

FIG. 49 is a flowchart of a process of outputting, in the virtual space 320, a tone or an image corresponding to the psychological safety level, performed by the information processing system 10 according to the present embodiment. The description with reference to FIG. 49 is focused on the differences from FIG. 15.

In step S1A, the user information acquisition unit 34 obtains the activity information of the avatars in the virtual space 320, and stores the activity information in time series for each avatar. The user group determination unit 32 extracts a group of avatars within a threshold distance from each other in the virtual space 320 (S 1A).

The user group determination unit 32 extracts a group of avatars whose directions are directed toward each other among the avatars within the threshold distance from each other (S2A).

Subsequent process of steps S3A to S7A can be similar to the process of steps S3 to S7 in FIG. 15. In the system configuration illustrated in FIG. 42, the image output in step S5A is displayed on the image display device 12 such as a laptop PC, for example, in front of the avatar. Alternatively, an image may be displayed in any desirable space by utilizing the merit of the virtual space 320. In the system configuration of FIG. 43, an image is displayed on the virtual image display device 12 in the virtual office 324. Alternatively, an image may be displayed in any desirable space by utilizing the merit of the virtual space 320. In either case, the image may or may not be visible to other avatars. Regarding the tone to be output, the tone may be output only to the display terminal 330 of the avatar whose psychological safety level is to be grasped, or may be output to the display terminals 330 of the users corresponding to all the avatars in the same virtual space 320.

Recording the transition of the psychological safety level between users (avatars) as illustrated in FIG. 16 is effective also in the virtual space 320.

### Creation of Minutes in which Conversation Partner is Identified

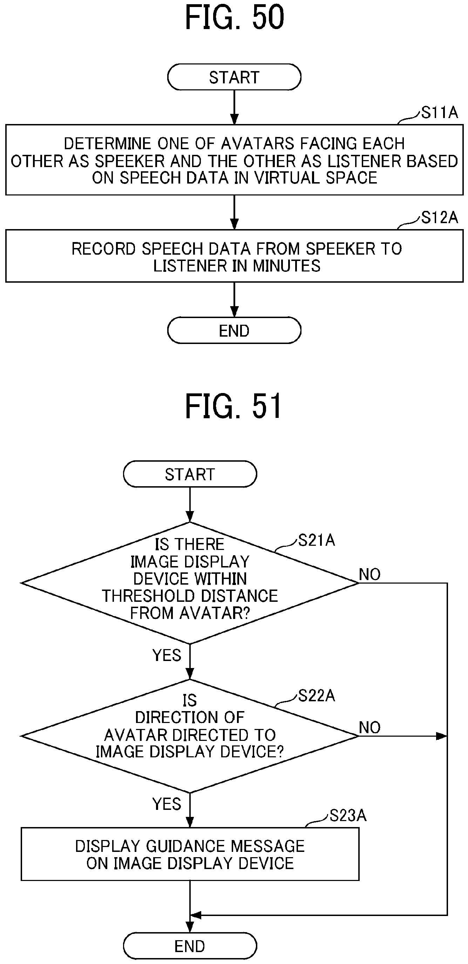

FIG. 50 is a flowchart of a process of creating minutes in which the conversation partner is identified in the virtual space 320, according to the present embodiment. The description with reference to FIG. 50 is focused on the differences from FIG. 22.

The minutes creation unit 39 determines one of the avatars facing each other is the speaker and the other is the listener based on speech data in virtual space 320 (S 11A). An utterance of an avatar is an utterance of a user in the real space. Since the activity sensor 9 transmits the speech data to the information processing system 10, the virtual space control unit 43 treats the speech data as that uttered by the avatar in the virtual space 320. The utterance may be transmission and reception of, for example, a chat or a stamp in the virtual space 320.

Then, the minutes creation unit 39 records, in the minutes 220, the speech data representing the utterance from the speaker to the listener (S 12A). The minutes creation unit 39 records the current psychological safety level in the minutes 220 in association with the speech data.

### Guidance Based on Avatar Position and Direction

FIG. 51 is a flowchart of a process of guiding an avatar to the image display device 12 in the virtual space 320, performed by the user guide unit 33 according to the present embodiment. The description with reference to FIG. 51 is focused on the differences from FIG. 25.

For example, every time the position of the avatar is detected in the virtual space 320, the user guide unit 33 determines whether there is the image display device 12 within the threshold distance from the avatar (S21A). In the system configuration of FIG. 42, since the avatar is in the virtual meeting room 321, the user guide unit 33 simply determines whether the image display device 12 such as a virtual display is present in front of the avatar. In the virtual space 320, however, an image can be displayed in any desirable space. Accordingly, the determination in step S21A may be omitted. In the system configuration of FIG. 43, since the avatar is in the virtual office 324 equivalent to the real office 325, the distance to the virtual image display device 12 in the virtual office 324 is determined. In the virtual space 320, however, an image can be displayed in any desirable space also in this case. Accordingly, the determination in step S21A may be omitted.

If the determination in step S21A is Yes, the user guide unit 33 determines whether the direction of the avatar is directed to the image display device 12 in the virtual space 320 (S22A).

In the system configuration of FIG. 42, since the avatar is in the virtual meeting room 321, the user guide unit 33 simply determines whether the direction of the avatar is directed to the image display device 12 such as a virtual display. Typically, in the virtual meeting room 321, the determined in step S22A is likely to be Yes. In the system configuration of FIG. 43, since the avatar is in the virtual office 324 equivalent to the real office 325, whether the avatar faces the virtual image display device 12 in the virtual office 324 is determined.

If the determination in step S22A is Yes, the second environmental control unit 37 displays a guidance message on the image display device 12 which the avatar faces (S23A). In the system configuration illustrated in FIG. 42, the guidance message is displayed on the image display device 12 such as a laptop PC, for example, in front of the avatar. Alternatively, the guidance message may be displayed in any desirable space by utilizing the merit of the virtual space 320. In the system configuration of FIG. 43, the guidance message is displayed on the virtual image display device 12 in the virtual office 324. Alternatively, the guidance message may be displayed in any desirable space by utilizing the merit of the virtual space 320. In either case, the message may or may not be visible to other avatars.

The guidance message is displayed, as illustrated in FIG. 26, voice input to the image display device 12 by the avatar is performed.

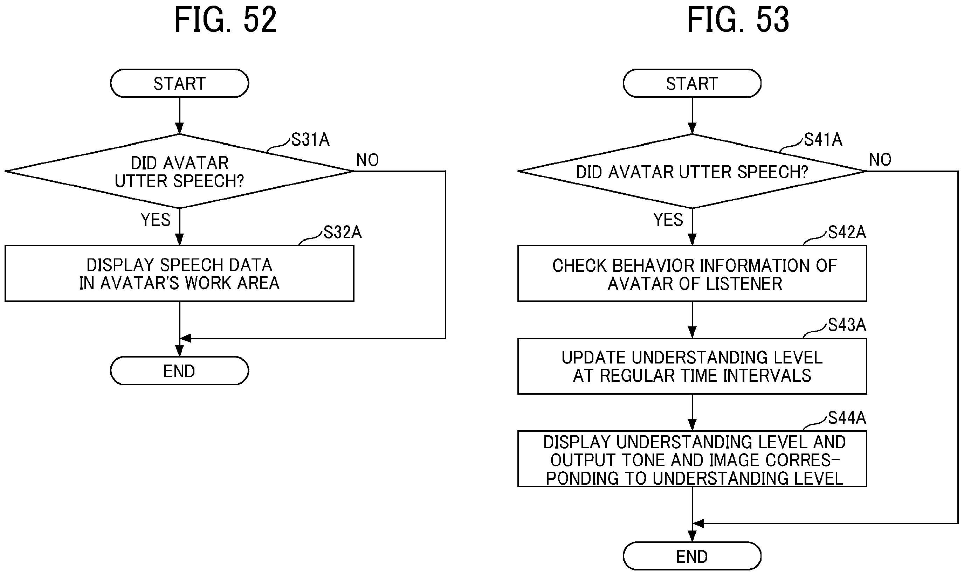

FIG. 52 is a flowchart of a process of displaying avatar's speech data in a work area, performed by the second environmental control unit 37 according to the present embodiment. The description with reference to FIG. 52 is focused on the differences from FIG. 27.

The second environmental control unit 37 determines whether the avatar has uttered words (S31A). Which avatar has uttered words is identified by the user ID transmitted by the activity sensor 9. The avatar's utterance is constantly recorded.

When the avatar utters words (Yes in S31A), the second environmental control unit 37 displays the speech data in the avatar's work area (S32A). As illustrated in FIG. 26, the work area of the avatar is an area dedicated to that avatar on the image display device 12. In the virtual space 320, the work area can be displayed in any desirable space, not limited to a wall. The work area may be a specific area set by the user or may be a preset area.

In the system configuration illustrated in FIG. 42 speech data is displayed on the image display device 12 such as a laptop PC, for example, in front of the avatar. Alternatively, speech data may be displayed in any desirable space by utilizing the merit of the virtual space 320. In the system configuration of FIG. 43, speech data is displayed on the virtual image display device 12 in the virtual office 324.

Alternatively, speech data may be displayed in any desirable space by utilizing the merit of the virtual space 320. In either case, the speech data may or may not be visible to other avatars.

### User State Determination

FIG. 53 is a flowchart of a process of displaying the understanding levels of avatars having a face-to-face conversation in the virtual space 320, performed by the second environmental control unit 37 according to the present embodiment. The description with reference to FIG. 53 is focused on the differences from FIG. 33.

The user state determination unit 41 determines whether the avatar has uttered a word (S41A). Which avatar has uttered words is identified by the user ID transmitted by the activity sensor 9. Further, another avatar facing the avatar who has uttered words is identified as the listener.

When the avatar utters words, the user state determination unit 41 checks the behavior information of the avatar being the listener with respect to the utterance (S42A). It is preferable that the user state determination unit 41 focuses on the behavior information of the avatar being the listener from the start to the end of the speech data and within a certain time from the end. The user state determination unit 41 records the behavior information together with time. The behavior information of the avatar is detected by the activity sensor 9. For example, when nodding or head tilt is detected as a behavior, the activity sensor 9 transmits the information on the detected behavior to the information processing system 10, and the virtual space control unit 43 treats the behavior as the behavior of the avatar in the virtual space 320. The behavior includes, in addition to the behavior detected by the activity sensor 9, behaviors (such as nodding and head tilt) input from the controller 331 operated by the user. Subsequent process of S43A and S44A can be similar to the process of S43 and S44 in FIG. 33.

In step S44A, in the system configuration illustrated in FIG. 42, the understanding level may be displayed on the image display device 12 such as a laptop PC, for example, in front of the avatar being the speaker.

Alternatively, the understanding level may be displayed in any desirable space by utilizing the merit of the virtual space 320. In the system configuration of FIG. 43, the understanding level may be displayed on the virtual image display device 12 in the virtual office 324. Alternatively, the understanding level may be displayed in any desirable space by utilizing the merit of the virtual space 320. In either case, the understanding level may or may not be visible to other avatars. Regarding the tone corresponding to the understanding level, the tone may be output only to the display terminal 330 of the user corresponding to the avatar being the speaker, or may be output to the display terminals 330 of the users corresponding to all the avatars in the same virtual space 320.

### Determination of Concentration Level

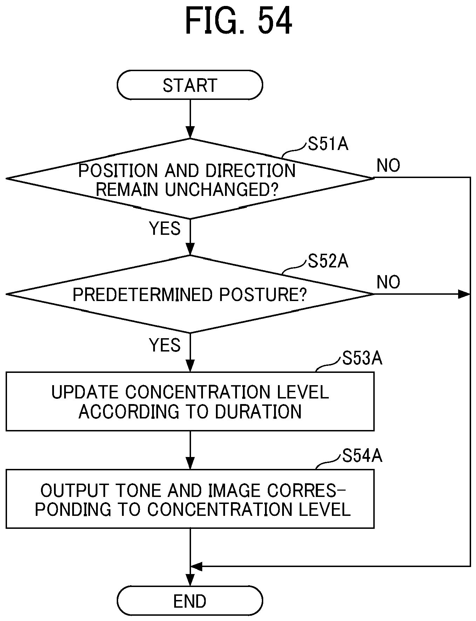

FIG. 54 is a flowchart of a process of environmental control in the virtual space 320 in accordance with the concentration level, according to the present embodiment. The description with reference to FIG. 54 is focused on the differences from FIG. 35.

The user state determination unit 41 determines whether the position and the direction of the avatar remain unchanged in the virtual space 320 (S51A).

When the determination in step S51A is Yes, the user state determination unit 41 determines whether the avatar is in a predetermined posture on the basis of the behavior information of the avatar (S52A). For example, when the behavior information indicates that the avatar is sleeping or doing exercises, the user state determination unit 41 determines that the avatar does not concentrate on work. The behavior information of the avatar is detected by the activity sensor 9. For example, when nodding or head tilt is detected as a behavior, the activity sensor 9 transmits the information on the detected behavior to the information processing system 10, and the virtual space control unit 43 treats the behavior as the behavior of the avatar in the virtual space 320. The behavior includes, in addition to the behavior detected by the activity sensor 9, behaviors (such as being interested and being concentrated) input from the controller 331 operated by the user. Subsequent process of S53A and S54A can be similar to the process of S53 and S54 in FIG. 35.

In step S54A, in the system configuration illustrated in FIG. 42, an image may be displayed on the image display device 12 such as a laptop PC, for example, in front of the avatar being the speaker. Alternatively, an image may be displayed in any desirable space by utilizing the merit of the virtual space 320. In the system configuration of FIG. 43, an image is displayed on the virtual image display device 12 in the virtual office 324. Alternatively, an image may be displayed in any desirable space by utilizing the merit of the virtual space 320. In either case, the image may or may not be visible to other avatars. Regarding the image corresponding to the concentration level, the image may be output only to the display terminal 330 of the user corresponding to the avatar being the speaker, or may be output to the display terminals 330 of the users corresponding to all the avatars in the same virtual space 320.

### Display of Cursor

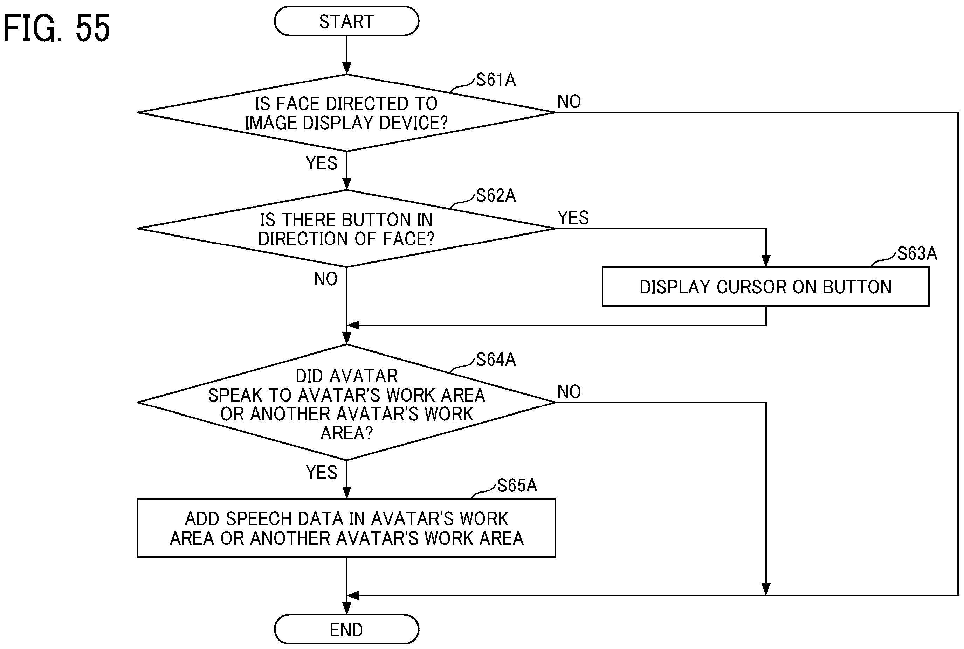

FIG. 55 is a flowchart of a method of displaying a cursor and usage of the cursor in the virtual space 320, according to the present embodiment. The description with reference to FIG. 55 is focused on the differences from FIG. 37.

The cursor position determination unit 42 determines whether the avatar faces the image display device 12 in the virtual space 320 (S61A). In the system configuration of FIG. 42, since the avatar is in the virtual meeting room 321, the user guide unit 33 simply determines whether the image display device 12 such as a virtual display is present in front of the avatar. In the virtual space 320, however, an image can be displayed in any desirable space. Accordingly, the determination in step S61A may be omitted. By contrast, on the premise that the cursor is to be moved to a button, since the button is displayed on the image display device 12, the determination of step S61A is performed. In the system configuration of FIG. 43, since the avatar is in the virtual office 324 equivalent to the real office 325, the distance to the virtual image display device 12 in the virtual office 324 is determined. In the virtual space 320, however, an image can be displayed in any desirable space also in this case. Accordingly, the determination in step S61A may be omitted. By contrast, on the premise that the cursor is to be moved to a button, since the button is displayed on the image display device 12, the determination of step S61A is performed.

When the determination in step S61A is Yes, the cursor position determination unit 42 determines whether there is a button in the direction of the avatars face (S62A).

If the determination in step S62A is Yes, the cursor position determination unit 42 determines to align the cursor position with the button (S63A). The second environmental control unit 37 displays a cursor superimposed on the button. The button may be in an area preset by the user. The button may be a button that is directly pressed by the user in the real space. By selecting or pressing the button, it is determined whether the avatar speaks toward the work area. Subsequent process of S64A and S65A can be similar to the process of S64 and S65 in FIG. 37.

### Team Evaluation Based on Utterance Content

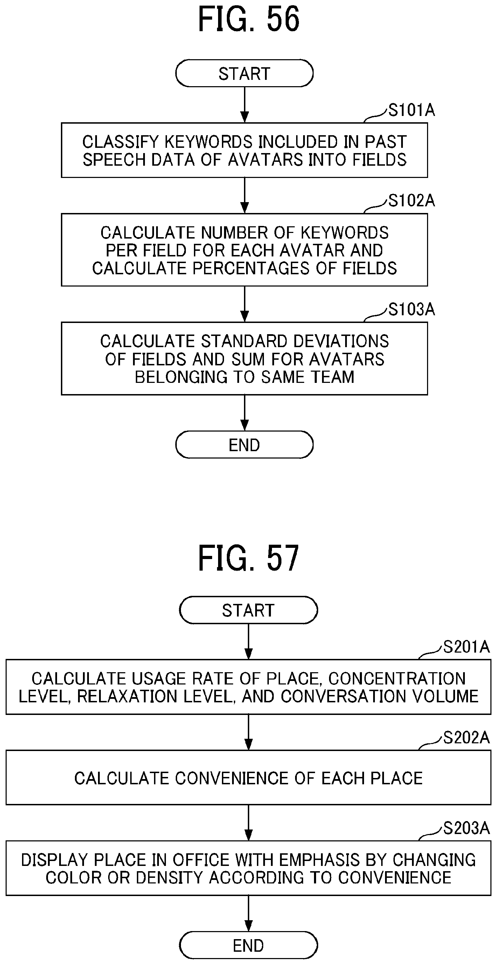

FIG. 56 is a flowchart of a process of scoring the individuality of a team in the virtual space 320, performed by the individuality evaluation unit 44 according to the present embodiment. The description with reference to FIG. 56 is focused on the differences from FIG. 29.

Also in the virtual space 320, since the speech data of the avatar is the speech data of the user, the process of S101A to 103A in FIG. 56 can be similar to the process of S101 to S103 in FIG. 29. Accordingly, even in the virtual space 320, the individuality evaluation unit 44 determines whether the individualities of members belonging to the same team are diversified.

### Office Optimization

FIG. 57 is a flowchart of a process of presenting the conveniences on an office layout, according to the present embodiment. The description with reference to FIG. 57 is focused on the differences from FIG. 39.

FIG. 57 is different from FIG. 39 in that the places are those in virtual space 320.

The process of S201A to S203A can be similar to the process of S201 to S203 in FIG. 39. In the system configuration of FIG. 42, the virtual space 320 can be designed as desired. Accordingly, the merit of optimizing the office layout is large.

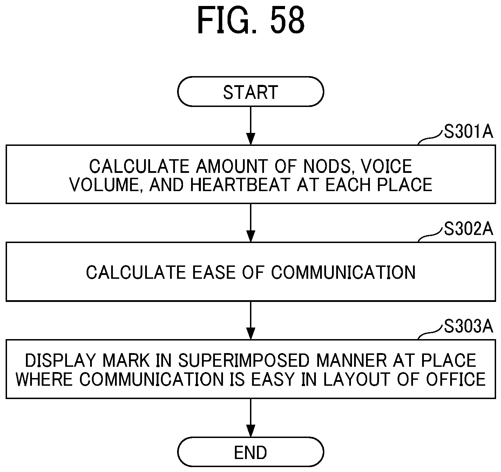

FIG. 58 is a flowchart of a process of presenting ease of communication on an office layout, according to the present embodiment. The description with reference to FIG. 58 is focused on the differences from FIG. 41.

FIG. 58 is different from FIG. 41 in that the places are those in virtual space 320.

The process of S301A to S303A can be similar to the process of S301 to S303 in FIG. 41. The avatars can communicate with each other by selecting an easy-to-communicate place in the virtual space 320.

### Applied Cases

The above-described embodiments are illustrative and do not limit the present disclosure. Thus, numerous additional modifications and variations are possible in light of the above teachings without deviating from the scope of the present disclosure. The information processing system 10 described in the above embodiments of the present disclosure is one example, and the system configuration may vary depending on applications or purposes.

For example, one user may carry multiple activity sensors 9. This makes it possible to detect the direction and behavior of the user more accurately.

The information processing system 10 may determine the direction and the behavior of the user by combining the direction and the behavior information from the activity sensor 9 and the movement of the user detected by the camera 18.

The information processing system 10 desirably performs active noise canceling on the sound data obtained by the activity sensor 9 using the sound data obtained by the microphone 20. Active noise canceling is to cancel ambient noise, and thus the user's voice can be obtained with reduced noise.

The functional configurations of, for example, FIG. 7 are illustrated in blocks divided according to functions in order to facilitate understanding of processing performed by the information processing system 10. No limitation is intended by how the processing units are divided by the unit of process or by the name. The processing units implemented by the information processing system 10 can be divided to a larger number of processing units depending on the contents of processing. Further, a single processing unit can be divided into multiple processing units.

Each of the functions of the above-described embodiments of the present disclosure may be implemented by one or more pieces of processing circuitry. The "processing circuit or circuitry" in the present specification includes a programmed processor to execute each function by software, such as a processor implemented by an electronic circuit, and devices, such as an application specific integrated circuit (ASIC), a digital signal processor (DSP), a field programmable gate array (FPGA), and conventional circuit modules arranged to perform the recited functions.

The present invention can be implemented in any convenient form within the scope of the appended claims, for example using dedicated hardware, or a mixture of dedicated hardware and software. The present invention may be implemented as computer software implemented by one or more networked processing apparatuses. The processing apparatuses include any suitably programmed apparatuses such as a general-purpose computer, a personal digital assistant, a Wireless Application Protocol (WAP) or third-generation (3G)-compliant mobile telephone, and so on. Since the present invention can be implemented as software, each and every aspect of the present invention thus encompasses computer software implementable on a programmable device. The computer software can be provided to the programmable device using any conventional carrier medium (carrier means). The carrier medium includes a transient carrier medium such as an electrical, optical, microwave, acoustic or radio frequency signal carrying the computer code. An example of such a transient medium is a Transmission Control Protocol/Internet Protocol (TCP/IP) signal carrying computer code over an IP network, such as the Internet. The carrier medium may also include a storage medium for storing processor readable code such as a floppy disk, a hard disk, a compact disc read-only memory (CD-ROM), a magnetic tape device, or a solid-state memory device.

The group of apparatuses or devices described in the embodiments of the present disclosure is one of multiple computing environments to implement the embodiments disclosed in the present disclosure. In some embodiments, the information processing system 10 includes multiple computing devices such as a server cluster. The multiple computing devices communicate with one another through any type of communication link including, for example, a network and a shared memory, and perform the processes disclosed in the present disclosure.

Further, the information processing system 10 can combine disclosed processing steps in various ways. The components of the information processing system 10 may be combined into a single apparatus or may be divided into multiple apparatuses. One or more of the processes performed by the information processing system 10 may be performed by the information processing terminal 22. Any one of the above-described operations may be performed in various other ways, for example, in an order different from the one described above.

The present disclosure further includes the following aspects.

In one aspect, the information processing system includes a user state determination unit to determine a state of the user based on the behavior information, and the environmental control unit controls the environment of the space in accordance with the state of the user.

In the information processing system of another aspect, the user state determination unit determines a concentration level as the state of the user in a case where a change in the position information and the direction information of the user is within a threshold; and the behavior information indicates that the user is in a predetermined posture. The environmental control unit controls the environment of the space in accordance with the concentration level of the user.

In the information processing system of another aspect, the user information acquisition unit acquires vital data of the user, and the environmental control unit controls the environment of the space in accordance with the position information, the direction information, and the vital data of the user.

In another aspect, the information processing system further includes a communication unit to receive speech data of the user, and the environmental control unit controls the environment of the space in accordance with the position information, the direction information, and the speech data of the user.

In another aspect, the information processing system further includes a user group determination unit to group multiple users in accordance with the position information and the direction information of the multiple users. The user state determination unit determines, as the state of the user, an understanding level of a conversation among the multiple users grouped, based on the behavior information, and the environmental control unit controls the environment of the space in accordance with the understanding level.

In another aspect, the information processing system further includes a cursor position determination unit to determine a position of a cursor to be displayed on an image display device in accordance with a face direction included in the behavior information of the user. The environmental control unit displays the cursor at a position determined by the cursor position determination unit, and the environmental control unit controls the environment of the space in accordance with the position of the displayed cursor and speech data of the user.

In the information processing system of another aspect, in a case where the cursor is displayed on a button, the environmental control unit controls the environment of the space in accordance with speech data of the user who presses the button.

In the information processing system of another aspect, the environmental control unit determines an object to be used for environmental control of the space in accordance with the position information and the direction information of the user, and displays a work area of the user on the determined object. In a case where a work area is present at the position determined as the position of the cursor by the cursor position determination unit, the environmental control unit additionally displays speech data of the user in the work area.

In another aspect, the information processing system further includes a psychological safety estimation unit to calculate a percentage of facing time to time during which a speaker and a listener belonging to a team and grouped by the user group determination unit have a conversation. The facing time is the duration in which the listener faces the speaker. The psychological safety estimation unit calculates a standard deviation of the percentage of the facing time in the team, and the environmental control unit controls the environment of the space in accordance with the standard deviation.

In another aspect, the information processing system further includes a psychological safety estimation unit to calculate a frequency of nodding of a listener to a speaker in time during which the speaker and the listener belonging to a team and grouped by the user group determination unit have a conversation; and calculate a standard deviation of the frequency of nodding in the team. The environmental control unit controls the environment of the space in accordance with the standard deviation.

In another aspect, the information processing system further includes an individuality evaluation unit to determine fields of keywords extracted from the speech data; calculate, for each speaker, percentages of the fields to which the keywords belong, and calculate a sum or an average of standard deviations of the percentages of the fields among speakers, as an individuality variation score of a team to which the speakers belong. The environmental control unit outputs the individuality variation score.

In another aspect, the information processing system further includes a convenience presentation unit to calculate, for each of multiple places in the space, a usage rate of the place where the user performs an activity, a concentration level of the user, a relaxation level of the user obtained from heartbeat intervals, and an conversation volume of the user obtained from speech data of the user, and present convenience of the place based on the usage rate of the place; and the concentration level, the relaxation level, and the conversation volume of the user.

In another aspect, the information processing system further includes a communicative level presentation unit to calculate, for each of multiple places in the space, an amount of nodding in an activity performed by the user at the place, a voice volume of utterances of the user at the place, and a heartbeat of the user at the place, and present ease of communication at the place calculated based on the amount of nodding, the sound volume, and the heartbeat of the user.

In another aspect, in the information processing system, the space includes a virtual space.

## Claims

1. An environmental control system for controlling an environment of a space in which a user performs an activity, the environmental control system comprising:
a user information acquisition unit (34) configured to receive position information and direction information of a user; and
an environmental control unit (36, 37) configured to control an environment of a space in which the user performs an activity, in accordance with the position information and the direction information of the user.

2. The environmental control system according to claim 1,
wherein the environmental control unit (36, 37) is configured to determine an object to be used for environmental control of the space in accordance with the position information and the direction information of the user.

3. The environmental control system according to claim 2,
wherein the environmental control unit (36, 37) is configured to display a work area of the user on the object determined in accordance with the position information and the direction information of the user.

4. The environmental control system according to claim 3, further comprising a communication unit (31) configured to receive speech data of the user,
wherein the environmental control unit (36, 37) is configured to display the speech data of the user in the work area of the user.

5. The environmental control system according to claim 3,
wherein the environmental control unit (36, 37) is configured to display a name of the user in the work area of the user.

6. The environmental control system according to any one of claims 1 to 5, further comprising a user group determination unit (32) configured to group multiple users in accordance with the position information and the direction information of the multiple users,
wherein the environmental control unit (36, 37) is configured to control the environment of the space in accordance with a state of the multiple users.

7. The environmental control system according to claim 6,
wherein the user group determination unit (32) is configured to group multiple users facing each other according to the position information and the direction information of the multiple users, and
wherein the environmental control system further comprises a user state determination unit (38) configured to determine the state of the grouped multiple users based on a time during which the grouped multiple users face each other.

8. The environmental control system according to claim 7,
wherein the state of the multiple users grouped includes a psychological safety level, and
wherein the environmental control unit (36, 37) is configured to control the environment of the space in accordance with the psychological safety level among the grouped multiple users.

9. The environmental control system according to claim 6,
wherein the user group determination unit (32) is configured to group multiple users facing each other according to the position information and the direction information of the multiple users, and
wherein the environmental control system further comprises a minutes creation unit (39) configured to record, in a memory, speech data of the multiple users together with information identifying a speech source and a speech destination among the multiple users.

10. The environmental control system according to claim 9,
wherein the minutes creation unit (39) is configured to record the speech data in association with group identification information of the grouped multiple users.

11. The environmental control system according to claim 9,
wherein the minutes creation unit (39) is configured to record the speech data together with the state of the multiple users grouped.

12. The environmental control system according to claim 1,
wherein the user information acquisition unit (34) is configured to receive behavior information of the user, and
wherein the environmental control unit (36, 37) is configured to control the environment of the space in accordance with the position information, the direction information, and the behavior information of the user.

13. The environmental control system according to any one of claims 1 to 12, further comprising:
an information processing apparatus (10) including the user information acquisition unit (34) and the environmental control unit (36, 37);
an activity sensor (9) carried by the user and configured to transmit the position information and the direction information of the user to the information processing apparatus (10); and
an output device (16; 22) including an output unit (92; 93) configured to output a tone or an image under control of the information processing apparatus (10).

14. The environmental control system according to claim 13, wherein the activity sensor (9) includes:
a sensor (705) configured to detect a position and a direction of the user; and
a transmission unit (61) configured to transmit, to the information processing apparatus (10), the position information indicating the position of the user detected by the sensor (705) and the direction information indicating the direction of the user detected by the sensor (705).

15. A carrier medium carrying computer readable codes which, when executed by a computer system, cause the computer system to carry out a method for controlling an environment of a space in which a user performs an activity, the method comprising
acquiring position information and direction information of a user in a space in which a user performs an activity; and
controlling an environment of the space in accordance with the position information and the direction information of the user.
